# EUROPEAN PATENT APPLICATION

(11) **EP 4 446 414 A1**
(43) Date of publication of application: **16.10.2024**
(21) Application number: 22903832.8
(22) Date of filing: 30.09.2022
(51) Int. Cl.: C12N 15/113, A61K 31/713, A61P 3/06, A61P 43/00, C12N 15/10

(54) **ANTISENSE OLIGONUCLEOTIDE COMPLEX**

(30) Priority: 07.12.2021 JP 2021198890
(71) Applicant: LIID Pharmaceuticals, Inc., Suita-shi, Osaka 564-8565 (JP)
(72) Inventor: YAMAMOTO, Tsuyoshi, Nagasaki-shi, Nagasaki 852-8521 (JP); TERADA, Chisato, Nagasaki-shi, Nagasaki 852-8521 (JP)
(74) Representative: J A Kemp LLP
(86) International application number: PCT/JP2022/036699
(87) International publication number: WO 2023/105898

(57) **Abstract**

The present invention provides an antisense oligonucleotide complex which includes an antisense oligonucleotide and a complementary strand which includes a sequence which is complementary to said antisense oligonucleotide, said complex being characterized in that said antisense oligonucleotide has a single strand-structured toe hold, and said complementary strand has nuclease resistance throughout the entire length thereof. As a result, said complex exhibits an antisense effect and has reduced toxicity.

## Description

### FIELD

The present invention relates to a novel antisense oligonucleotide complex and use thereof, and to a method for producing the nucleic acid and the like.

### BACKGROUND

One active area in the recent ongoing development of drugs known as "nucleic acid drugs" involves development based on the antisense method, which allows high selectivity of target genes, etc. A variety of artificial nucleic acids have been developed and introduced, and their formulations marketed, with the goal of enhancing the nuclease resistance of antisense oligonucleotides (ASO) and improving binding affinity and specificity for target nucleic acids.

With increasing experience of use of antisense nucleic acids in the clinic, however, the problem of how to avoid the latent toxicity of antisense oligonucleotides has been recognized as an obstacle against clinical use and wider application of antisense nucleic acids. One cause of toxicity with antisense oligonucleotides is so-called "off-target toxicity". The sources of off-target toxicity are largely divided into two categories (NPL 1). These are: toxicity due to binding of ASO having the same or similar sequences as target RNA (hybridization-dependent toxicity), and toxicity due to the higher-order structure or physicochemical properties of ASO (hybridization-independent toxicity). However, it is still unknown to how these two mechanisms mutually contribute to the mechanism of toxicity that is exhibited.

NPL 2, etc. discloses a method that can reduce hybridization-dependent toxicity wherein the number of non-naturally occurring nucleotides of ASO is adjusted to minimize its binding strength with target RNA. However, such reduction in binding strength is often associated with lowering of the drug effect. NPL 3 discloses a method that can reduce toxicity in which one non-natural nucleic acid is introduced into the gap region of gapmer ASO. In this method, however, it is impossible to predict the resulting changes in activity and toxicity, making the method poorly suitable for wide use.

In addition, DNA/RNA heteroduplex oligonucleotides (HDO) have been developed as nucleic acids using the antisense method (PTL 1, NPL 4). HDO consists of a main chain that binds to target mRNA and has antisense activity, and an RNA (cRNA) strand that is complementary to the main chain, with the cRNA strand being cut by the intracellular endonuclease RNase H at the double stranded center section. The isolated main chain hybridizes with the target mRNA as a result, exhibiting a gene expression-regulating effect (NPL 4). The cRNA also functions for efficient transport of the main chain (ASO) to target cells, and it has been reported that HDO can be efficiently delivered to the liver by bonding of the drug delivery ligand molecule tocopherol to the cRNA strand (PTL 1, NPL 5).

PTL 1 also teaches the use of peptide nucleic acid (PNA) in place of the RNA strand of the DNA/RNA heteroduplex oligonucleotide in order to facilitate binding of functional molecules such as peptides to the double-stranded nucleic acid complex. However, in PTL 1 the PNA is not used from the viewpoint of lowering toxicity of double-stranded nucleic acid, and nothing is mentioned regarding the actual toxicity of double-stranded nucleic acids with PNA.

The inventors of PTL 1 have reported that administration of ASO to subjects as a nucleic acid complex with a double-stranded structure has low toxicity in the central nervous system (PTL 2). Specifically, PTL 2 states that toxicity is lowered when using RNA or DNA as the complementary strand for ASO. PTL 2 also states that HDO with overhang on the complementary strand (also known as overhanging-duplex oligonucleotide, ODO) has an *in vivo* gene suppressing effect in mice, but it does not mention whether HDO without overhang on the complementary strand retains its activity. One scientific article on ODO (NPL 6) teaches that HDO without overhang exhibited almost no activity *in vivo.* In other words, in order for HDO to exhibit an adequate antisense effect it is thought necessary for an overhang to be attached to the complementary strand.

### [CITATION LIST]

### [PATENT LITERATURE]

[PTL 1] International Patent Publication No. WO2013/089283
[PTL 2] International Patent Publication No. WO2019/181946

### [NON PATENT LITERATURE]

[NPL 1] Pharmaceutical and Medical Device Regulatory Science PMDRS, 51(2), 70-82(2020); Yokota, T. (2021), Jikken Igaku, Special Edition, Vol.39, No.17, Yodosha, 145-156
[NPL 2] Hagedorn et al., Nucleic Acids Res, 46:5366-5380 (2018)
[NPL 3] Nature Biotechnology volume 37:640-650 (2019)
[NPL 4] Kuwahara H. et al., Sci Rep. 8(1):4377 (2018)
[NPL 5] Molecular Therapy, 29(2):838-847 (2021)
[NPL 6] Mon S et al., FEBS Letters 594:1413-1423 (2020)

### SUMMARY

### [TECHNICAL PROBLEM]

It is an object of the present invention to provide a novel antisense oligonucleotide complex with reduced toxicity while having antisense oligonucleotide activity, as well as a pharmaceutical composition comprising the antisense oligonucleotide complex. It is another object of the invention to provide a method for lowering toxicity while having antisense oligonucleotide activity, and a method for producing the antisense oligonucleotide complex.

### [SOLUTION TO PROBLEM]

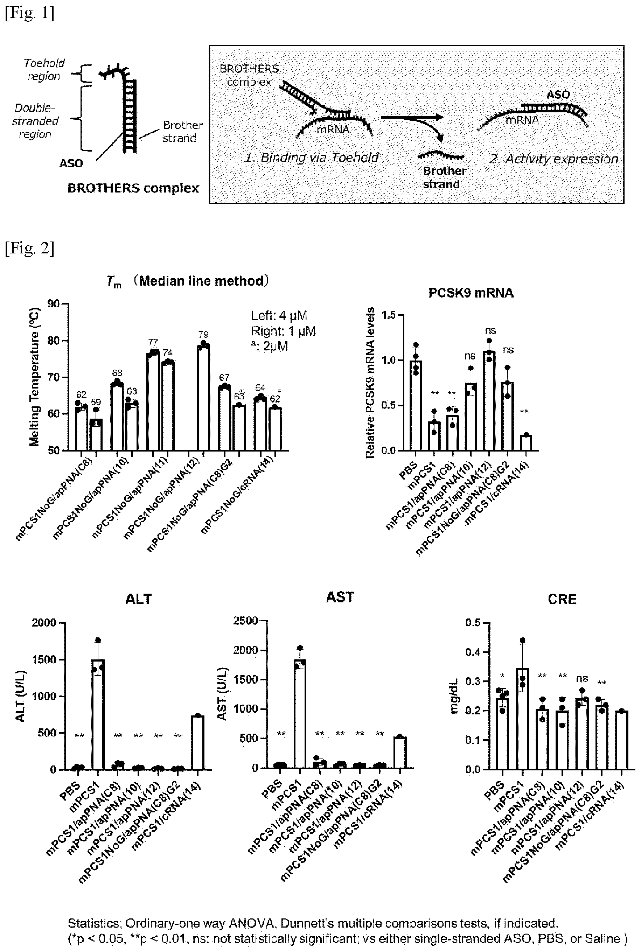

The present inventors have devised a novel double-stranded nucleic acid having a complementary toehold, which is complementary to part of the target RNA at the 5'-end and/or 3'-end of the antisense strand, as a means for simultaneously solving the problems of hybridization-dependent and non-dependent toxicity. Specifically, the idea was that part of the ASO was covered with a complementary strand to construct a toehold region on the ASO. It was conjectured that the ASO and target RNA engage in highly target-selective weak binding via the toehold region, forming a 3-dimensional construct comprising a target RNA-antisense strand-complementary strand, followed by removal of the complementary strand to form a target RNA-antisense nucleic acid construct, thereby allowing the antisense effect to be exhibited (see also Fig. 1).

In this double-stranded nucleic acid, ASO is rectified by the complementary strand to have a partial double helix structure, thereby imparting a well-controlled uniform higher-order structure and physicochemical properties regardless of the nucleotide sequence, so that it is expected to allow reduction in hybridization-independent toxicity. The present inventors gave the double-stranded antisense nucleic acid having this structure the name "BROTHERS" (Brace on Therapeutic ASO), and named the complementary strand the "Brother strand" or "Bracing strand".

Since BROTHERS exists as a double-strand up until binding of ASO to its target RNA, and binds to the target via the toehold, the *in vivo* stability of the brother strand is especially important. This type of so-called "strand-exchange reaction" is known to be accelerated 10⁶-fold with double-stranded nucleic acid having a toehold, as compared to full-matching double-stranded nucleic acid (J. Am. Chem. Soc. 2009, 131, 47, 17303-17314). Strand-exchange reaction via a toehold is known to be inefficient in the presence of mismatching, but it can be appropriately designed so as to maximize mismatch recognition (Biophysical Journal, 2016, 110, 7, 1476-1484; J. Am. Chem. Soc. 2020, 142, 11451-11463). It was conjectured that this principle could be applied to antisense oligonucleotides to avoid hybridization-dependent toxicity as well.

Based on these facts, the brother strand may have as its complementary strand a nucleic acid such as PNA that has high binding strength with ASO without being metabolized by nucleases, and also having low interaction with biomolecules that are responsible for toxicity. On the other hand, it is known that in order for an HDO to exhibit an antisense effect, it is necessary for the complementary strand to be efficiently cleaved by unspecified nucleases to expose ASO, and that replacing the complementary strand with nucleic acid that is not a substrate for nucleases prevents the antisense effect from being exhibited (Yokota, T. (2021) Jikken Igaku Special Edition, Vol.39, No.17, Yodosha: 37-43). The BROTHERS of the present invention is a completely different invention from previous HDOs in light of the type of toxicity to be reduced, the mechanism by which the effect is exhibited and specifications regarding the structure and design of the complementary strand.

By using BROTHERS for single-stranded ASO which is known to exhibit hepatotoxicity, the present inventors were able to succeed in significantly inhibiting the hepatotoxicity of ASO. Surprisingly, in different BROTHERS complexes having brother strands of different lengths, a correlation was found between the binding strength of the double-strand and the antisense effect and toxicity. Specifically, it was found that gradually increasing double-stranded binding strength results in gradually lower hepatotoxicity, and also a gradually attenuated antisense effect. By optimizing the balance between double-stranded nucleic acid binding strength, and/or toehold-target RNA binding strength, and/or ASO-target RNA binding strength, it was shown that it can be to lower toxicity alone while maintaining the antisense effect. Interestingly, the knockdown activity of ASO was completely lost with full-matching double-stranded nucleic acid lacking the toehold. This was also found to occur with ASO for different target mRNAs. An improved effect on nephrotoxicity was also found. Next-generation sequence analysis showed that the response of gene groups producing oxidative stress (ROS) thought to be associated with tissue damage and gene groups that reduce ROS, as well as the response of gene groups associated with cell death, were inhibited in the BROTHERS complex compared to single-stranded ASO. It was also demonstrated that using the BROTHERS complex lowered mitochondrial toxicity. This strongly suggested that the BROTHERS complex has low toxicity not only for the liver and kidneys but also for general tissues and organs in the body. Also surprisingly, it was found that binding of targeting molecules to ASO itself can efficiently inhibit expression of genes in target cells. Conversely, according to one embodiment the antisense activity was markedly attenuated in target organs when targeting molecules were bound to the brother strand that exhibits nuclease resistance across the full length. The present inventors conducted further research based on this finding, which led to completion of the present invention.

Specifically, the invention provides the following.
[1-1] An antisense oligonucleotide complex which comprises an antisense oligonucleotide and a complementary strand comprising a sequence complementary to the antisense oligonucleotide,
   wherein the antisense oligonucleotide has a toehold with a single-stranded structure, and
   the complementary strand has nuclease resistance across its full length,
   the antisense oligonucleotide complex having an antisense effect and reduced toxicity.
[1-2] The antisense oligonucleotide complex according to [1-1], wherein the complementary strand does not comprise a region with 4 or more consecutive natural nucleotides.
[1-3] The antisense oligonucleotide complex according to [1-1] or [1-2], wherein the toxicity is hepatotoxicity and/or nephrotoxicity.
[2] The antisense oligonucleotide complex according to any one of [1-1] to [1-3], wherein the length of the toehold is 1 to 10 bases in length.
[3-1] The antisense oligonucleotide complex according to any one of [1-1] to [2], wherein all of the nucleotides composing the complementary strand are non-natural nucleotides.
[3-2] The antisense oligonucleotide complex according to any one of [1-1] to [3-1], wherein all of the internucleotide in the complementary strand are modified.
[4] The antisense oligonucleotide complex according to any one of [1-1] to [3-2], wherein at least one of the nucleotides composing the complementary strand is a PNA nucleotide.
[5] The antisense oligonucleotide complex according to any one of [1-1] to [4], wherein all of the nucleotides composing the complementary strand are PNA nucleotides.
[6] The antisense oligonucleotide complex according to any one of [1-1] to [5], wherein:
   (1) the melting temperature (Tm) of the double-stranded nucleic acid is 40°C to 80°C, and/or
   (2) the length of the complementary strand is 35% to 95% of the length of the antisense oligonucleotide.
[7-1] The antisense oligonucleotide complex according to any one of [1-1] to [6], wherein:
   (1) the Tm is 50°C to 70°C, and/or
   (2) the length of the complementary strand is 60% to 80% of the length of the antisense oligonucleotide.
[7-2] The antisense oligonucleotide complex according to any one of [1-1] to [7-1], wherein the length of the antisense strand is 7 to 35 bases in length.
[7-3] The antisense oligonucleotide complex according to any one of [1-1] to [7-2], wherein the antisense strand is a gapmer oligonucleotide or mixmer oligonucleotide.
[8] The antisense oligonucleotide complex according to any one of [1-1] to [7-3], wherein at least one functional molecule is bonded to either or both the antisense oligonucleotide strand and complementary strand.
[9-1] The antisense nucleotide complex according to [8], wherein the functional molecule is bonded only to the antisense oligonucleotide.
[9-2] The antisense nucleotide complex according to [9-1], wherein at least one functional molecule is a targeting molecule.
[9-3] The antisense nucleotide complex according to any one of [8] to [9-2], wherein at least one functional molecule other than a targeting molecule is bonded to either or both the antisense strand and complementary strand.
[10] The antisense nucleotide complex according to any one of [1] to [9-3], wherein the antisense oligonucleotide targets mRNA selected from the group consisting of PCSK9 mRNA, ApoB mRNA, AcsL1 mRNA and ApoC3 mRNA.
[11-1] A pharmaceutical composition comprising an antisense oligonucleotide complex according to any one of [1-1] to [10].
[11-2] The pharmaceutical composition according to [11-1], which is for treatment or prevention of a disease.
[11-3] The composition according to [11-1] or [11-2], which is for treatment or prevention of a disease while reducing toxicity.
[11-4] The composition according to any one of [11-1] to [11-3], which is for subcutaneous administration.
[11-5] The composition according to any one of [11-1] to [11-4], wherein the disease is dyslipidemia, diabetes, liver disease or kidney disease.
[12-1] A composition for regulating expression of a target gene, comprising an antisense oligonucleotide complex according to any one of [1-1] to [10].
[12-2] The composition according to [12-1], wherein the regulation of expression is suppression of expression.
[13] A method for producing an antisense oligonucleotide complex with reduced toxicity, comprising:
   (1) a step of preparing an antisense oligonucleotide,
   (2) a step of preparing a complementary strand in which the antisense oligonucleotide has a toehold with a single-stranded structure when a double-strand has been formed with the antisense oligonucleotide, and wherein
      the complementary strand has nuclease resistance across the full length, and
   (3) a step of annealing the antisense oligonucleotide prepared in step (1) and the complementary strand prepared in step (2).
[14] The method according to [13], wherein the length of the toehold is 1 to 10 bases in length.
[15] A method for designing an antisense oligonucleotide complex with reduced toxicity, comprising:
   (1) a step of selecting an antisense oligonucleotide, and
   (2) a step of designing a complementary strand in which the antisense oligonucleotide has a toehold with a single-stranded structure when a double-strand has been formed with the antisense oligonucleotide, and wherein
   the complementary strand has nuclease resistance across the full length.
[16] The method according to [15], wherein the length of the toehold is 1 to 10 bases in length.
[17] The method according to any one of [13] to [16], wherein the complementary strand does not comprise a region with 4 or more consecutive natural nucleotides.
[18] The method according to any one of [13] to [17], wherein the complementary strand further has the following characteristics when the complementary strand has formed a double-strand with the antisense oligonucleotide:
   (1) the melting temperature (Tm) of the double-stranded nucleic acid is 40°C to 80°C, and/or
   (2) the length of the complementary strand is 35% to 95% of the length of the antisense oligonucleotide.
[19] The method according to any one of [13] to [18], wherein all of the nucleotides composing the complementary strand are non-natural nucleotides.
[20] The method according to any one of [13] to [19], wherein at least one non-natural nucleotide is a PNA nucleotide.
[21] A method for treating or preventing a disease in a mammal, comprising administering an effective amount of the antisense oligonucleotide complex according to any one of [1-1] to [10] into the mammal.
[22] The antisense oligonucleotide complex according to any one of [1-1] to [10] for use in the treatment or prevention of a disease.
[23] Use of the antisense oligonucleotide complex according to any one of [1-1] to [10] in the manufacture of an agent for treating or preventing a disease.
[24] A method for regulating expression of a target gene, comprising administering an effective amount of the antisense oligonucleotide complex according to any one of [1-1] to [10] into a cell or a mammal.
[25] The antisense oligonucleotide complex according to any one of [1-1] to [10] for use in the regulation of expression of a target gene.
[26] Use of the antisense oligonucleotide complex according to any one of [1-1] to [10] in the manufacture of a composition for regulating expression of a target gene.

### [ADVANTAGEOUS EFFECTS OF INVENTION]

According to the invention it is possible to devise novel double-strand nucleic acids and produce the nucleic acids, and also to reduce the toxicity of existing nucleic acids. Such double-strand nucleic acids may exhibit lower toxicity than single-stranded nucleic acids and HDO, and are therefore particularly useful for drug purposes.

### BRIEF DESCRIPTION OF DRAWINGS

Fig. 1 shows an overview of the structure and mechanism of BROTHERS (Brace on Therapeutic ASO).
Fig. 2 shows measurement results for double-stranded nucleic acid melting temperature (Tm), PCSK9 mRNA expression level, ALT activity level, AST activity level and CRE amount, when antisense oligonucleotide (ASO) or double-strand nucleic acid was subcutaneously administered to mice. Error bar: standard deviation (SD)
Fig. 3 shows in-vivo dynamics imaging and systemic *ex vivo* fluorescent images after intravenously administering fluorescent-labeled antisense oligonucleotide (ASO) without ligand or double-strand nucleic acid with ligand on the complementary strand side to mice through the caudal vein.
Fig. 4 shows visualization results for bound proteins for single-stranded ASO or BROTHERS.
Fig. 5 shows measurement results for double-stranded nucleic acid melting temperature (Tm), apolipoprotein B (ApoB) mRNA expression level, ALT activity level and CRE amount, when antisense oligonucleotide (ASO) or double-strand nucleic acid was subcutaneously administered to mice. Error bar: SD
Fig. 6 shows measurement results for double-stranded nucleic acid melting temperature (Tm), ApoB mRNA expression level, ALT activity level and CRE amount, when antisense oligonucleotide (ASO) or double-strand nucleic acid was subcutaneously administered to mice. Error bar: SD
Fig. 7 shows the results of introducing antisense oligonucleotide (ASO) or double-strand nucleic acid exhibiting toxicity into human liver cancer cells (HepG2) and measuring Caspase 3/7 activity. Error bar: SD
Fig. 8 shows the results of transcriptome analysis and Pathway analysis of gene expression in liver tissue, when single-stranded ASO or BROTHERS was subcutaneously administered to mice.
Fig. 9 shows time-dependent change in target PCSK9 mRNA expression level, body weight and ALT activity level when single-stranded ASO or BROTHERS was subcutaneously administered once to mice. Error bar: SD
Fig. 10 shows the results of measuring cell viability and deviant enzyme LDH activity in culture medium, after introducing single-stranded ASO exhibiting *in vivo* toxicity or double-strand nucleic acid exhibiting no toxicity into human liver cancer cells (Huh-7). Error bar: SD
Fig. 11 shows comparative photographs of intracellular dynamics using a confocal microscope, after introducing fluorescent-labeled single-stranded ASO exhibiting *in vivo* toxicity or double-strand nucleic acid exhibiting no toxicity into human liver cancer cells (Huh-7).
Fig. 12 shows visualization results for bound proteins for single-stranded ASO or BROTHERS.
Fig. 13 shows measurement results for ALT activity after double-strand nucleic acid obtained using a complementary strand with similar structure to that known for HDO as a complementary strand was subcutaneously administered to mice. Error bar: SD
Fig. 14 shows evaluation of cell viability, effect on mitochondria and Caspase 3/7, 9. 8 activities, after introducing single-stranded ASO exhibiting *in vivo* toxicity and double-strand nucleic acid exhibiting no toxicity into human liver cancer cells (Huh-7). Error bar: SD
Fig. 15 shows *in vitro* evaluation of strand-exchange reaction rate for a mismatch gene, based on change in fluorescence intensity. Shown are the results of comparing strand-exchange reaction rate and degree of off-target knockdown in actual cells (Huh-7). Error bar: SD
Fig. 16 shows experimental results in mice, demonstrating effectively reduced side-effects when parallel PNA was used in constructing BROTHERS having suitable binding strength for longer single-stranded ASO and a suitable coverage ratio (ratio of double-stranded region to strand length). Error bar: SD
Fig. 17 shows, based on measurement of the double-stranded nucleic acid melting temperature (Tm), that the stability of a BROTHERS duplex can be adjusted and optimized by introducing a functional molecule such as an amino acid at the end. Error bar: SD
Fig. 18 shows target gene knockdown activity when reacting human liver cancer cells (Huh-7) with brother strands having adjusted lengths and relative positions on the antisense strand, as well as lengths, positions and number of toeholds. Error bar: SD

### DESCRIPTION OF EMBODIMENTS

### 1. Antisense oligonucleotide complex

The present invention provides an antisense oligonucleotide complex (hereunder also referred to as "complex of the invention") having an antisense oligonucleotide (also referred to as "antisense strand"), and a complementary strand (also referred to as "brother strand") comprising a sequence complementary to the full or partial sequence of the antisense oligonucleotide (also referred to as "double-strand-forming region"), which may be referred to as the "complementary sequence". The complex of the invention may be one consisting of an antisense oligonucleotide and a complementary strand (i.e. a double-strand nucleic acid), or it may have another substance such as a functional molecule covalently bonded or non-covalently bonded to the double-strand nucleic acid. According to one aspect, the double-strand nucleic acid of the complex of the invention is characterized in that (1) the double-stranded nucleic acid melting temperature (Tm) is within a specified range, and/or (2) the length of the complementary strand is a length with a specified ratio with respect to the length of the antisense oligonucleotide. The antisense oligonucleotide composing the complex of the invention has a toehold in at least one from among the 5'-end, 3'-end and center section, while the complementary strand composing the complex of the invention has resistance against nucleases such as RNase H. Such resistance against nucleases is typically conferred to the complementary strand by ensuring that it does not include a region with 4 or more consecutive natural nucleotides. If the double-strand nucleic acid of the complex of the invention has such a structure it can exhibit an antisense effect on target RNA and also attenuated toxicity, even without using the double-stranded nucleic acid binder as described in PTL 2. As used herein, the terms "comprising" or "has" include the concept of "consisting of", unless otherwise specified.

As used herein, the term "antisense strand" or "antisense oligonucleotide" means a single-stranded oligonucleotide exhibiting an antisense effect. The antisense oligonucleotide composing the complex of the invention comprises a double-strand-forming region and a toehold, the double-stranded-forming region integrating with the toehold to form an antisense region, and the antisense region participating in hybridization with part of the target RNA (hereunder also referred to as "target region"). That is, the sequence of the antisense region is complementary to the sequence of the target region (hereunder also referred to as "target sequence"). As used herein, "toehold" is used to mean a single-stranded structure comprising part of the target sequence, as a "toehold" for hybridization between the antisense oligonucleotide and the target RNA. The terms "strand-exchange reaction" and "inversion" indicates a reaction in which one nucleic acid strand of the nucleic acids forming a double-strand is completely or partially replaced with a different nucleic acid strand, forming a new complex.

As used herein, "nucleic acid" may refer to a monomer nucleotide, but it usually refers to an oligonucleotide comprising multiple monomers. Unless otherwise specified, therefore, the term "nucleic acid nucleotide" will be used for monomer nucleotides, examples of such nucleic acids including ribonucleic acid, deoxyribonucleic acid, peptide nucleic acid (PNA) and morpholino nucleic acid. Unless otherwise specified, when a nucleic acid is an oligonucleotide, the nucleotide residues composing the nucleic acid (including the 5'-end and 3'-end nucleotides) will be referred to simply as "nucleotides". The terms "nucleic acid strand" and "strand" used herein both refer to single-stranded oligonucleotides, unless otherwise specified. A nucleic acid strand can be created as a full length strand or partial strand by a chemical synthesis method (such as a method using an automatic synthesizer) or an enzymatic process (including, but not limited to, polymerase, ligase and restriction reaction).

As used herein, "antisense activity" means activity of hybridizing with target RNA, the result of the hybridization being an antisense effect (for example, regulated expression of a gene, such as reduced target RNA amount, or reduced or increased level of protein translated from target RNA). Examples of target RNA for the complex of the invention include mRNA, noncoding RNA (ncRNA) (e.g. miRNA, etc.) or exogenous RNA (e.g. viral RNA, etc.), with mRNA being preferred. The term "mRNA" used herein includes not only mature mRNA but also mRNA without base modification, unprocessed mRNA precursors (pre-mRNA), and so on. The "target gene" whose expression is to be regulated by the antisense effect is not particularly restricted and may be a gene from an organism in which the complex of the invention is to be transferred, such as a gene whose expression is increased or decreased in various diseases, for example. Examples of specific target mRNAs include, but are not limited to, PCSK9 mRNA, ApoB mRNA, AcsL1 mRNA and ApoC3 mRNA. As used herein, the phrase "expression of a gene" is used to include the concept of "production of a transcription product such as mRNA or miRNA encoded by the gene", while also including "production of a functional protein encoded by the target mRNA".

Specifically, when the target is PCSK9 mRNA, the complex of the invention may be a combination of any of the antisense strands listed in Table 1 and Table 2 (mPCS1, mPCSINoG or mPCS1NoGaf) and any of the brother strands listed in Table 1 and Table 2 (apPNA(12)-mPCS1, apPNA(11)-mPCS1, apPNA(10)-mPCS1, apPNA(C8)-mPCS1 or apPNA(C8)G2-mPCS 1). When the target is ApoB mRNA, for example, it may be a combination of any of the antisense strands listed in Table 4, Table 5 and Table 8 (GN6, hApo1 or hApoB-521-BNA (13); hApo1), with any of the brother strands listed in Table 4, Table 5 and Table 8 (apPNA(12)-GN6, apPNA(11)-GN6, apPNA(10)-GN6, apPNA(9)-GN6, apPNA(8)-GN6, apPNA(C8)-GN6, apPNA(C5)-hApo1, apPNA(C6)-hApo1, apPNA(C7)-hApo1, apPNA(C8)-hApo1, apPNA(C9)-hApo1, apPNA(C10)-hApo1, apPNA(C11)-hApo1, apPNA(C12)-hApo1 or apPNA(C13)-hApo1). When the target is AcsL1 mRNA, for example, it may be a combination of any of the antisense strands listed in Table 7 (Acsl1, Acsl1n or Acsl1b) with any of the brother strands listed in Table 7 (apPNA(C14)-Acsl1, apPNA(C15)-Acsl1, apPNA(C16)-Acsl1, apPNA(C17)-Acsl1 or apPNA(C18)-Acsl1). When the target is ApoC3 mRNA, for example, it may be a combination of the antisense strand listed in Table 9 (hAPOC3-AS) with any of the brother strands listed in Table 9 (3-0PNA(6), 3-0PNA(7), 3-0PNA(8), 3-0PNA(9), 3-0PNA(10), 3-1PNA(6), 3-1PNA(7), 3-2PNA(7), 3-2PNA(8), 5-0PNA(6), 5-0PNA(7), 5-1PNA(7), 5-2PNA(7) or 5-2PNA(8)). Of the brother strands listed in Table 9, 3-0PNA(6), 3-1PNA(6), 3-1PNA(7), 3-2PNA(7), 5-0PNA(6), 5-0PNA(7), 5-1PNA(7), 5-2PNA(7) and 5-2PNA(8) are preferred, and 3-1PNA(7), 5-0PNA(6), 5-0PNA(7), 5-1PNA(7), 5-2PNA(7) and 5-2PNA(8) are more preferred. For Tables 1, 2, 4, 5 and 7 to 9, when a targeting molecule such as GalNAcₕₚ or GalNAc_{apd} is added to the end of a sequence, a complex without the aforementioned molecules added, or with any targeting molecule added in any number (such as 1 to 4), may be used. Even if a targeting molecule is not added to the end of the sequence, a complex with any targeting molecule added in any number (such as 1 to 4) may be preferably used.

The antisense effect can be measured, for example, by administering a test nucleic acid compound to a subject (such as a mouse), and after several days to several months (for example, after 2 to 7 days or after 1 month), for example, measuring the expression level of the target gene or the level of target transcription product whose expression is to be regulated by antisense activity provided by the test nucleic acid compound (for example, the mRNA level or the amount of RNA such as microRNA, the amount of cDNA, the protein level and so on).

As used herein, "exhibit an antisense effect" means that the expression level of a target gene or the level of a target transcription product which is to be measured decreases (or increases) (a decrease (or increase) of 10%, 20%, ≥30%, 40%, 50% or more, for example) in at least one arbitrary experimental system, compared to a negative control (for example, administration of a nucleic acid-free vehicle).

When the antisense oligonucleotide forms a double-stranded region with target RNA and the double-stranded region is cut by a ribonuclease (such as RNase H), the target RNA level is decreased. Alternatively, when the antisense oligonucleotide forms a double-stranded region with target mRNA and translation by ribosomes is inhibited, expression of the protein encoded by the mRNA is inhibited at the translation level. An antisense oligonucleotide that exhibits such an effect may be referred to hereunder as a "suppression type antisense oligonucleotide".

The antisense oligonucleotide forms a double-stranded region with the splicing promoting sequence of pre-mRNA, masking the sequence and causing the splicing to be skipped, and thereby increasing the abundance of the functional protein in cells. Alternatively, it may form a double-stranded region with a binding sequence for an enzyme that degrades the mRNA, which is present in the target mRNA, thus masking the sequence and suppressing degradation of the mRNA. An antisense oligonucleotide that exhibits such an effect may be referred to hereunder as an "enhancement type antisense oligonucleotide".

The suppression type antisense oligonucleotide is preferably a gapmer oligonucleotide, from the viewpoint of stably and efficiently cleaving RNA in cells or the body. According to the invention, "gapmer oligonucleotide" refers to a chimeric antisense oligonucleotide wherein an internal region (also referred to herein as "gap region") having multiple (such as 5, 6, 7, 8, 9, 10, 11, 12, 13, 14, 15 or more) nucleotides (typically deoxyribonucleotides) recognized by RNase H is situated between external regions (the 3'-end external region may be referred to as the "3' wing region" and the 5'-end external region may be referred to as the "5' wing region") having one or more (e.g.: 1, 2, 3, 4, 5, 6, 7, 8, 9, 10 or more) nucleotides modified so as to confer resistance to ribonucleases. At least one nucleotide forming each region, the 3' wing region and 5' wing region, is preferably a non-natural nucleotide. A non-natural nucleotide is preferably a nucleotide with a crosslinked structure (hereunder also referred to as bridged nucleic acid: BNA).

From the viewpoint of stability in cells and in the body, the enhancement type antisense oligonucleotide preferably has at least one nucleotide modified so as to confer resistance to nucleases such as RNase H. Such an antisense oligonucleotide may be one in which all of the nucleotides are non-natural nucleotides, or one in which some of the nucleotides are non-natural nucleotides (i.e. a mixmer oligonucleotide). Such non-natural nucleotides are preferably bridged nucleic acids.

The target region in the target RNA is not particularly restricted so long as it is a region in the RNA, but includes, for example, 3'UTR, 5'UTR, an exon, intron, protein coding region, translation initiation region, translation termination region, another nucleic acid region and so on.

As used herein, "complementary" means that the nucleic acid bases are in a relationship allowing formation of Watson-Crick base pairs (naturally-occurring base pairs or non-Watson-Crick base pairs (such as Hoogsteen base pairs or wobble base pairs), via hydrogen bonding. The term "complementary sequence" is therefore used in the sense which includes not only sequences that are completely complementary (hybridizing without mismatches) to the sequence of interest (such as the complementary sequence in the complementary strand or the sequence of the target region in target RNA), but also sequences containing one or more (such as 2, 3, 4, 5 or more) mismatches, so long as they can hybridize to the target sequence under stringent conditions or under physiological conditions of mammalian cells. In other words, mismatches are allowed in the complementary strand. For example, sequences having 80% or higher (e.g., 85% or higher, 90% or higher, 95% or higher, 96% or higher, 97% or higher, 98% or higher, 99% or higher) identity, most preferably 100% identity, to a sequence that is completely complementary to the target sequence are included. The Tm and strand exchange efficiency can be adjusted by introducing mismatches into the antisense oligonucleotide and its complementary strand, and by the number of mismatches. For a specific aspect in which the complementary strand is a PNA strand, the double-strand nucleic acid may be not only the anti-parallel form but also the parallel form. The aforementioned definition of "complementary" is therefore applied not only to the relationship between each nucleic acid strand of anti-parallel double-strand nucleic acids, but also to the relationship between each nucleic acid strand of parallel double-strand nucleic acids.

The stringent conditions may be low stringent conditions or highly stringent conditions. Low stringent conditions may be conditions of relatively low temperature and high salt concentration, such as 30°C, 2 × SSC, 0.1% SDS. Highly stringent conditions may be conditions of relatively high temperature and low salt concentration, such as 65°C, 0.1 × SSC, 0.1% SDS. By varying the temperature and salt concentration conditions it is possible to adjust the hybridization stringency. The reference to 1 × SSC includes 150 mM sodium chloride and 15 mM sodium citrate.

The length of the antisense region may usually be, but is not particularly limited to, at least 7 bases in length, at least 8 bases in length, at least 9 bases in length, at least 10 bases in length, at least 11 bases in length, at least 12 bases in length or at least 13 bases in length. The antisense region in the antisense oligonucleotide may be 35 bases in length or less, 30 bases in length or less, 25 bases in length or less, 24 bases in length or less, 23 bases in length or less, 22 bases in length or less, 21 bases in length or less, 20 bases in length or less, 19 bases in length or less, 18 bases in length or less, 17 bases in length or less or 16 bases in length or less. The antisense region in the antisense strand oligonucleotide may be 7 to 35 bases in length, 7 to 30 bases in length, 7 to 25 bases in length, 7 to 20 bases in length, 8 to 20 bases in length, 9 to 20 bases in length, 10 to 20 bases in length, 11 to 18 bases in length or 12 to 16 bases in length.

The length of the antisense oligonucleotide is not particularly restricted, but may be at least 7 bases in length, at least 8 bases in length, at least 9 bases in length, at least 10 bases in length, at least 11 bases in length, at least 12 bases in length or at least 13 bases in length. The antisense oligonucleotide may be 50 bases in length or less, 45 bases in length or less, 40 bases in length or less, 35 bases in length or less, 30 bases in length or less, 28 bases in length or less, 26 bases in length or less, 24 bases in length or less, 22 bases in length or less, 20 bases in length or less, 18 bases in length or less or 16 bases in length or less. The antisense oligonucleotide may be 9 to 50 bases in length, 10 to 40 bases in length, 11 to 35 bases in length or 12 to 30 bases in length, for example.

The length of the complementary strand is not particularly restricted, but it preferably satisfies either or both the following conditions when the complementary strand and antisense oligonucleotide have formed a double strand: (1) a length such that the double-stranded nucleic acid melting temperature (Tm) is within a specified range, and (2) a length which is a specific ratio with respect to the length of the antisense oligonucleotide. Specifically, the length of the complementary strand may usually be, but is not particularly limited to, at least 7 bases in length, at least 8 bases in length, at least 9 bases in length, at least 10 bases in length, at least 11 bases in length, at least 12 bases in length or at least 13 bases in length. The complementary strand may be 50 bases in length or less, 45 bases in length or less, 40 bases in length or less, 35 bases in length or less, 30 bases in length or less, 28 bases in length or less, 26 bases in length or less, 24 bases in length or less, 22 bases in length or less, 20 bases in length or less, 18 bases in length or less or 16 bases in length or less. The complementary strand may be 9 to 50 bases in length, 10 to 40 bases in length, 11 to 35 bases in length or 12 to 30 bases in length, for example.

The phrase "double-strand nucleic acid melting temperature" as used herein means the temperature at which 50% of the oligonucleotide in solution forms double-strands with the completely complementary molecules, while the remaining 50% is free in solution. Reference herein to a specific melting temperature is the melting temperature measured by the following method. The final concentration is measured after heating a sample solution (150 µL) containing 10 mM phosphate buffer (pH 7.0), 100 mM sodium chloride, 0.1 mM ethylenediaminetetraacetic acid and 4 µM of each oligonucleotide (antisense oligonucleotide and complementary strand) at 95°C for 3 minutes and then slowly cooling to 20°C at a rate of 1°C/min for annealing. The absorbance at 260 nm is plotted at 1°C intervals while heating to 90°C at 0.5°C per minute. All of the Tₘ values are calculated by the median line method. The decimal places are rounded off. Depending on the type of nucleic acid, it may not be possible to measure the melting temperature under the aforementioned conditions due to problems of absorbance size or solubility. In such cases, the Tm value used is that measured under reasonable conditions approximating the conditions described above, with slight adjustment of the concentration conditions with addition of a salt or organic solvent.

The specific range for the double-strand nucleic acid melting temperature is preferably 40°C or higher (such as 45°C, 50°C, 51°C, 52°C, 53°C, 54°C, 55°C, 56°C, 57°C, 58°C, 59°C, 60°C, 61°C, 62°C, 63°C, 64°C or higher), and preferably 80°C or lower (such as 77°C, 75°C, 74°C, 73°C, 72°C, 71°C, 70°C, 69°C, 68°C, 67°C or lower). The melting temperature of the double-strand nucleic acid of the complex of the invention may be 40°C to 80°C, 50°C to 70°C, 40°C to 77°C, 40°C to 73°C, 51°C to 77°C, 52°C to 67°C, 62°C to 72°C, 62°C to 68°C, 64°C to 72°C or 64°C to 79°C.

The specific ratio for the length of the complementary strand is preferably 35% or higher (such as 38%, 39%, 40%, 45%, 50%, 51%, 52%, 53%, 54%, 55%, 56%, 57%, 58%, 59%, 60%, 61%, 62% or higher) and preferably 95% or lower (such as 94%, 92%, 90%, 85%, 84%, 83%, 82%, 81%, 80%, 79%, 78%, 77%, 76%, 75%, 74%, 73%, 72%, 71%, 70%, 69% or lower), with respect to the length of the antisense oligonucleotide. The length of the complementary strand may be 35% to 95%, 60% to 80%, 38% to 92%, 54% to 92%, 57% to 79% or 57% to 71% with respect to the length of the antisense oligonucleotide. The ratio is calculated as: (number of bases of complementary strand/number of bases of antisense oligonucleotide) × 100, rounding off the decimal places.

The toehold may be present at the 5'-end, at the 3'-end, at the center section or at both ends of the antisense oligonucleotide. The toehold may also be formed by metabolism of part of the complementary strand in the body. The length of each toehold may be 1 base in length or longer, or at least 1 base in length, at least 2 bases in length, at least 3 bases in length, at least 4 bases in length or at least 5 bases in length. The upper limit for the length of the toehold is not particularly restricted but may be 15 bases in length or less, 14 bases in length or less, 13 bases in length or less, 12 bases in length or less, 11 bases in length or less, 10 bases in length or less, 9 bases in length or less, 8 bases in length or less, 7 bases in length or less or 6 bases in length or less. The length of the toehold may be 1-15 bases in length, 1-10 bases in length, 1-7 bases in length, 1-6 bases in length, 3-5 bases in length or 4-5 bases in length.

As mentioned above, the complementary strand composing the complex of the invention exhibits resistance to nucleases. Nucleases including RNase H are enzymes that catalyze hydrolysis of phosphodiester bonds between nucleosides of complementary strands forming a DNA/RNA heteroduplex and/or DNA/DNA heteroduplex. The phrase "having nuclease resistance across the full length" means that the aforementioned hydrolysis is not catalyzed in any of the phosphodiester bonds in the complementary strand forming the nucleic acid, by various nucleases (at least RNase H) in cells (such as mammalian cells) into which the complex of the invention to be introduced. For example, if the complementary strand does not comprise a region with 4 or more consecutive natural nucleotides, it is possible to confer nuclease resistance across the full length of the complementary strand. According to a preferred aspect, therefore, the complementary strand does not comprise a region with 4 or more consecutive natural nucleotides, and the phrase "has nuclease resistance across the full length" may be interpreted as meaning "does not comprise a region with 4 or more consecutive natural nucleotides". While the phrase "exhibits nuclease resistance" does not exclude degradation by ribonucleases other than RNase H, the strand is preferably not hydrolyzed by other ribonucleases such as RNase A or DNase I, from the viewpoint of intracellular stability.

In order for the complementary strand composing the complex of the invention to not be a substrate to nucleases, at least one nucleotide composing the complementary strand may be a non-natural nucleotide, at least one bond between nucleosides of the complementary strand may be a non-phosphodiester bond, a non-natural molecule may be bonded as a cap at the end of the complementary strand, or a combination of these may be employed as appropriate. Such a combination can ensure that the complementary strand does not comprise a region with 4 or more consecutive natural nucleotides. According to one aspect of the invention, all of the nucleotides composing the complementary strand are non-natural nucleotides, or all of the internucleoside bonds in the complementary strand are non-phosphodiester bonds. Examples of non-phosphodiester bonds (hereunder also referred to as "modified internucleoside bonds") include, but are not limited to, phosphorothioate bonds, phosphorodithioate bonds, phosphotriester bonds, methyl phosphonate bonds, methylthiophosphonate bonds, boranophosphate bonds, phosphoroamidate bonds and so on. One type of non-natural nucleotide or modified internucleoside bond may be used, or several types may be used in combination.

As used herein, "non-natural nucleotide" means a nucleotide other than a natural nucleotide, or a nucleotide analog, in which at least one of the components of a natural nucleotide (a ribonucleotide or deoxyribonucleotide) is modified, and it may also be referred to as "modified nucleotide". Components of nucleotides include sugars (such as ribose and deoxyribose), bases and phosphoric acid. The term "modification" includes, for example, a substitution, addition and/or deletion of the component and/or internucleoside bond, and a substitution, addition and/or deletion of an atom and/or functional group of the component and/or internucleoside bond.

Natural nucleotides include adenine, cytosine, guanine, thymine and uracil. Examples of modified bases in which the base is modified include but are not limited to 5-methylcytosine, 5-fluorocytosine, 5-bromocytosine, 5-iodocytosine or N4-methylcytosine; N6-methyladenine or 8-bromoadenine; and N2-methylguanine or 8-bromoguanine. A modified base is preferably 5-methylcytosine.

Examples of modifications of sugar portion include 2'-O-methoxyethyl modification of the sugar portion, 2'-O-methyl modification of the sugar portion, 2'-fluoro modification of the sugar portion, bridging of the 2'-position and 4'-position of the sugar portion (nucleotides with crosslinked structures are BNA), and so on. Examples of BNA include locked nucleic acid (LNA), 2'-O,4'-C-ethylene bridged nucleic acid (ENA: 2'-O,4'-C-Ethylene bridged Nucleic Acid), and so on. The following nucleoside structures may be mentioned as more specific BNA.

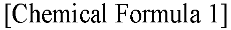

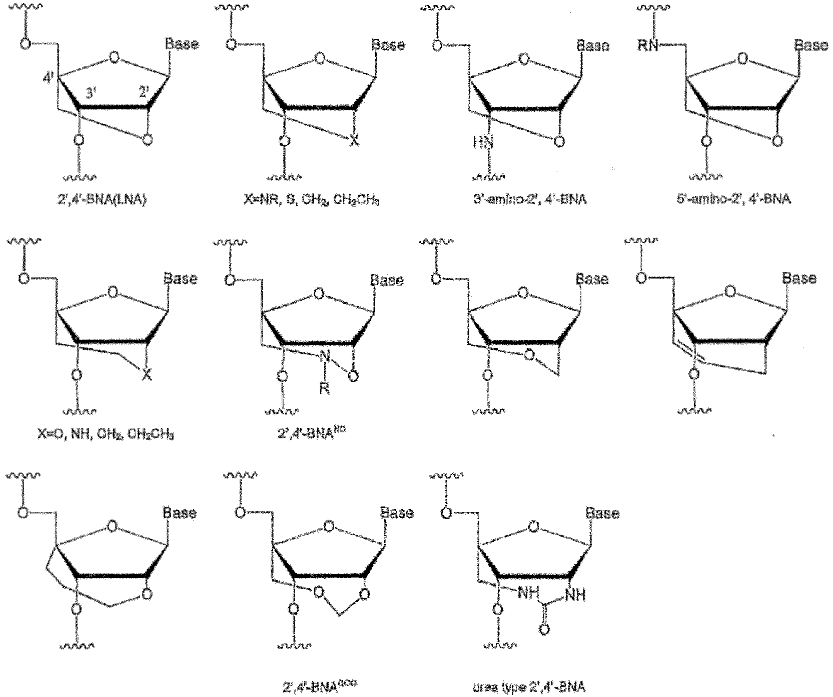

(In the formulas, R represents a hydrogen atom, an optionally branched or cyclic alkyl group of 1 to 7 carbon atoms, an optionally branched or cyclic alkenyl group of 2 to 7 carbon atoms, an aryl group of 3 to 12 carbon atoms optionally including a heteroatom, an aralkyl with an aryl group portion of 3 to 12 carbon atoms optionally including a heteroatom, or an amino group protecting group for nucleic acid synthesis. Preferably, R is a hydrogen atom or a methyl, ethyl, n-propyl, isopropyl, phenyl or benzyl group, and more preferably R is a hydrogen atom or a methyl group. The Base is a natural base or modified base.)

Examples of nucleotide analogs include peptide nucleic acids (PNA), gamma-PNA (γ-substituted PNA), morpholino nucleic acids (-N(H)-P(=O)(-NR₁R₂)-O- [where R₁ and R₂ are both methyl groups] or other non-phosphodiester bonded morpholino), boranophosphate-type nucleic acid (-O-P(-BH₃)(=0)-O-), acyclic threoninol nucleic acid (aTNA), serinol nucleic acid (SNA), acyclic glycol nucleic acid (GNA) and tricyclo-DNA. PNA is a nucleotide analog having a main chain with N-(2-aminoethyl)glycine bonded by an amide bond instead of a sugar, and the following are PNA structures in which all of the nucleotides (n + 2 nucleotides) are PNA nucleotides. The Tm of the double-strand can be adjusted by appropriate introduction of a chiral unit such as an amino acid at the N-terminus or C-terminus of the PNA chain. According to one embodiment, at least one nucleotide of complementary strand composing the complex of the invention is a PNA nucleotide, and more preferably all of the nucleotides of the complementary strand are PNA nucleotides.

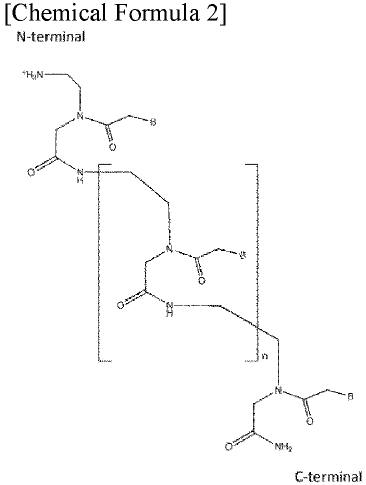

(In the formula, B is a natural base or modified base.)

The following is a morpholino nucleic acid nucleotide.

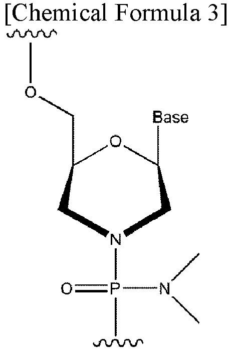

(In the formula, Base is a natural base or modified base.)

The following is a boranophosphate-type nucleic acid nucleotide.

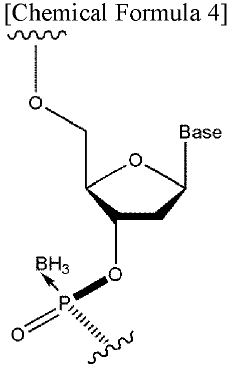

### (In the formula, Base is a natural base or modified base.)

As mentioned above, the complex of the invention having the specific structure can exhibit antisense activity for target RNA with low toxicity. As used herein, "toxicity" means an effect causing undesirable objective or subjective symptoms or dysfunction in a subject, such as death, pain, tremor, cramp, motor disability, cognitive function impairment, impaired consciousness, general malaise, fatigue, nausea or vomiting, dizziness, numbness or light-headedness. The toxicity may be toxicity in any organ (such as the liver or kidneys), of a nature causing dysfunction and/or functional decline in the organ. The complex of the invention may have at least low hepatotoxicity, and preferably also low nephrotoxicity. As used herein, the terms "lowered toxicity" or "low toxicity" mean that toxicity is low or absent based on as specific marker when the complex of the invention has been administered to cells or the body, compared to when a control nucleic acid with toxicity has been administered under the same conditions. The complex can be evaluated to have low toxicity when low toxicity is found in at least one arbitrary test system. For hepatotoxicity, the AST activity level or ALT activity level in serum may be used as the indicator of toxicity, for example. For nephrotoxicity, the amount of CRE in serum may be used as the indicator of toxicity, for example. The control nucleic acid may be an antisense oligonucleotide composing the complex of the invention or an antisense oligonucleotide composing HDO. The term "has toxicity" means higher toxicity than a negative control (for example, administration of a vehicle that does not comprise nucleic acid).

Each nucleic acid of the complex of the invention may also be bonded with one or more (1, 2, 3, 4 or more) functional molecules. Such a functional molecule may be bonded at the 5'-end and/or 3'-end of the antisense oligonucleotide, or bonded at the 5'-end and/or 3'-end of the complementary strand, or bonded to both the antisense oligonucleotide and complementary strand. When two or more functional molecules are present, they may be bonded at different locations of the oligonucleotide, or bonded in tandem. Bonding between the nucleic acid strand and functional molecule of the complex of the invention may be direct bonding or indirect bonding via a different substance. However, the functional molecule is preferably directly bonded to the oligonucleotide by covalent bonding, ionic bonding or hydrogen bonding, and more preferably covalent bonding from the viewpoint of allowing more stable bonding to be achieved. The functional molecule may also be bonded to the oligonucleotide via a cleavable linking group. For example, the functional molecule may be linked by a disulfide bond. Moreover, only one type of functional molecule may be used, or a plurality of types may be used in combination

The structure of the functional molecule is not particularly restricted so long as it can confer the desired function to each nucleic acid of the complex of the invention. Examples of desired functions include a labeling function, a purifying function and a delivery function. Examples of substances that confer a labeling function include compounds such as fluorescent proteins and luciferase. Examples of substances that confer a purifying function include compounds such as biotin, avidin, His-tag peptide, GST tag peptide and FLAG tag peptide. The functional molecule may be one that changes the nature of the double-strand nucleic acid (for example, its Tm, its ability to bind with the target sequence, etc.) by bonding with double-strand nucleic acid, and specifically, for example, it may be a Tm-regulating substance such as a natural or non-natural amino acid or chiral low molecular compound (e.g., a drug such as ibuprofen, etc.).

According to one embodiment, the functional molecule performs the role of enhancing transport to target cells or cell nuclei, the term "targeting molecule" or "drug delivery molecule" being used to refer to a functional molecule that performs such a role. For example, certain peptide tags have been shown to enhance uptake of oligonucleotides into cells when conjugated with the oligonucleotides. The targeting molecule used for the invention may therefore be arginine-rich peptide P007 or B peptide which are disclosed in HaiFang Yin et al., Human Molecular Genetics, Vol. 17(24), 3909-3918 (2008) and its references, for example. Nuclear transport can be enhanced by conjugating a substance such as m3G-CAP (see Pedro M. D. Moreno et al., Nucleic Acids Res., Vol. 37, 1925-1935 (2009)) with a oligonucleotide. Moreover, only one type of targeting molecule may be used, or a plurality of types may be used in combination

The targeting molecule may also be an amino sugar. Examples of amino sugars include N-acetylgalactosamine (GalNAc), and more specifically GalNAc ligand having a (2S,4R)-4-hydroxy-L-prolinol backbone (GalNAcₕₚ), GalNAc ligand having a 3-amino-1,2-propanediol backbone (GalNAc_{apd}) (Bioorg. Med. Chem, 2016,24,26, Nucleos Nucleot Nucl, 2020, 39,109, Curr Protoc Nucleic Acid Chem, 2019, 78, e99. Nucleic Acid Therapeutics, Doi: 10.1089/nat.2021.0036) and so on. The targeting molecule may also be a lipid. Examples of lipids include lipids such as cholesterol and fatty acids (such as vitamin E (tocopherol, tocotrienol), vitamin A and vitamin D); fat-soluble vitamins such as vitamin K (for example, acylcarnitine); intermediate metabolites such as acyl-CoA; glycolipids, glycerides, and their derivatives or analogs. Cholesterol or vitamin E (tocopherol and tocotrienol) are preferred among these from the viewpoint of higher safety. Water-soluble vitamins such as folic acid may also be used. From the viewpoint of allowing the nucleic acid to be delivered with high specificity and high efficiency by binding to the various proteins present on the cell surfaces of different organs (e.g., receptors), the targeting molecule is preferably a low molecular ligand, aptamer, peptide or protein (for example, a receptor ligand or antibody and/or its fragment).

The nucleic acid to which the functional molecule is bonded may be produced by carrying out synthesis, purification and annealing by a publicly known method using a nucleic acid species to which the functional molecule is already bonded. Numerous methods for linking functional molecules to nucleic acids are known in the technical field. For example, when using GalNAc as the functional molecule, a GalNAc amidite block may be used in the phosphoroamidite method for linking to the nucleic acid. The GalNAc amidite block can be synthesized by the method described in Curr Protoc Nucleic Acid Chem. 2019, 78, e99. Doi: 10.1002/cpnc.99., 2. Nucleic Acid Ther. 2021, in press. Doi: 10.1089/nat.2021.0036., etc. for example. Alternatively, the nucleic acid strand can be ordered and acquired from a manufacturer (such as GeneDesign, Inc.), specifying the base sequence and modification site or type.

As demonstrated by the Examples below, targeting molecules were found to have high antisense effects even when bonded only to the antisense oligonucleotide. The targeting molecules are therefore preferably bonded only to the antisense oligonucleotide. When the targeting molecule is bonded only to the antisense oligonucleotide, a functional molecule other than the targeting molecule may be bonded to the complementary strand.

The following double-strand nucleic acids (1) and/or (2) may be excluded from among double-strand nucleic acids for the complex of the invention. PNA nucleic acids listed in the Sequence Listing have their sequences shown left to right from the N-terminus to the C-terminus.

### [Chemical Formula 5]

Double-strand nucleic acid (1)
5'-GCattggtatTC-3' (SEQ ID NO: 37)
C'- UAACCAUAAG-N' (SEQ ID NO: 38)
5'-GCattggtatTC-3'
C'- ACCAUAAG-N'
(uppercase: LNA, lowercase: DNA, underlined: PNA)

### [Chemical Formula 6]

Double-strand nucleic acid (2)
5'-GCattggtatTC-3'
C'- TAACCATAAG-N' (SEQ ID NO: 39)
5'-GCattggtatTC-3'
C'- ACCATAAG-N'
(uppercase: LNA, lowercase: DNA, underlined: PNA)

### 2. Use of complex of the invention

As mentioned above, the complex of the invention can regulate expression of its target gene by its antisense activity. The complex of the invention can therefore be used as a composition for regulating expression of a target gene expression (or an agent for regulating expression of a target expression) (hereunder this may also be referred to as "composition of the invention"). The composition of the invention is a composition having reduced toxicity (for example, low hepatotoxicity or low nephrotoxicity) of the antisense oligonucleotide. The term "composition for regulating gene expression" encompasses both the concept of "composition for suppressing gene expression" and "composition for enhancing gene expression". The composition of the invention may be prepared as a cosmetic or food and administered orally or parenterally. The composition of the invention may also be used as a reagent or test agent.

When the composition of the invention includes two or more nucleic acids, or when it includes another reagent type, the composition may be provided as a composition kit that includes each nucleic acid and reagent type in separate compositions.

The composition of the invention may be administered to a subject as the complex of the invention alone, or together with a pharmacologically acceptable carrier. Examples of the subject for introduction, for example, includes mammalian animals including a human, and cells, tissue or organs of the animals, etc. Also provided, therefore, is a method for regulating expression of a gene in a subject comprising administrating the complex of the invention into the subject.

In order to promote introduction of the complex of the invention into the target cells, the composition of the invention may further include a reagent for nucleic acid introduction. The reagent for nucleic acid introduction may be calcium chloride, a calcium enrichment reagent, atelocollagen, liposomes, nanoparticles, or a cationic lipid such as lipofectin, lipofectamine, DOGS (transfectam), DOPE, DOTAP, DDAB, DHDEAB, HDEAB, polybrene or poly(ethyleneimine) (PEI).

Since the complex of the invention has low toxicity as mentioned above, it is especially suitable for use as a drug. Also provided, therefore, is a pharmaceutical composition comprising the complex of the invention (also referred to hereunder as "pharmaceutical composition of the invention"). The pharmaceutical composition of the invention can be used for the treatment or prevention of a disease, and may also be used for the treatment or prevention of a disease while reducing toxicity of the antisense oligonucleotide. According to the invention, the term "therapeutic" encompasses the concept of alleviating or improving symptoms, and preventing, delaying or stopping progression of a disease or symptoms or manifestation of symptoms. Examples of such diseases include diseases associated with overexpression or excessive accumulation of a protein encoded by target mRNA of the complex of the invention, or deficiency or reduction in a protein encoded by target mRNA of the complex of the invention. Examples of such diseases include, but are not limited to, dyslipidemia such as hypercholesterolemia and atherosclerosis (which are treatable by suppressing expression ofPCSK9, ApoB or ApoC3, for example), diabetes (which is treatable by suppressing expression of AcsL1, for example), liver disease and kidney disease, and so on.

The pharmaceutical composition of the invention may consist of an effective amount of the complex of the invention alone, or it may be formulated together with an optional carrier such as a pharmacologically acceptable carrier.

Examples of pharmacologically acceptable carriers include excipients such as sucrose and starch, binders such as cellulose and methyl cellulose, disintegrators such as starch and carboxymethyl cellulose, lubricants such as magnesium stearate and Aerosil, aromatic agents such as citric acid and menthol, preservatives such as sodium benzoate and sodium bisulfite, stabilizers such as citric acid and sodium citrate, suspending agents such as methyl cellulose, and polyvinylpyrrolidone, dispersing agents such as surfactants, diluents such as water and physiological saline, and base waxes, with no limitation to these.

In order to promote introduction of the complex of the invention into the target cells, the pharmaceutical composition of the invention may further include a reagent for nucleic acid introduction. The reagent for nucleic acid introduction used in this case may be any of the same ones mentioned above.

The pharmaceutical composition of the invention may also be a pharmaceutical composition obtained by encapsulating the complex of the invention in liposomes. Liposomes are microscopic closed vesicles having an inner phase surrounded by one or more lipid bilayers, normally with a water-soluble substance held in the inner phase and a fat-soluble substance in the lipid bilayer. When "encapsulation" is used herein, the complex of the invention may be held in the inner phase of the liposomes, or that it is held in a lipid bilayer. The liposomes to be used for the invention may be a monolayer film or multilayer film, with particle diameters appropriately selected in the range of 10 to 1000 nm and preferably 50 to 300 nm, for example. In consideration of deliverability to target tissues, the particle diameters are 200 nm or smaller and preferably 100 nm or smaller, for example.

The method selected for encapsulating a water-soluble compound such as an oligonucleotide in liposomes may be any publicly known method without limitations, such as a lipid film method (vortex method), reversed-phase evaporation method, surfactant removal method, freezing-thawing method or remote loading method.

The pharmaceutical composition of the invention may be administered perorally or parenterally, to a mammal (e.g.: human, rat, mouse, guinea pig, rabbit, sheep, horse, pig, cow, dog, cat or monkey), but it is preferably administered parenterally. Therefore, also provided is a method for treating or preventing a disease in a mammal, comprising administering an effective amount of the complex of the invention into the mammal.

Suitable formulations for parenteral administration (such as subcutaneous injection, intramuscular injection, intravenous infusion, local infusion (topical external use or topical application), intracerebroventricular administration, intrathecal administration or intraperitoneal administration) include aqueous and nonaqueous isotonic aseptic injections, which may include antioxidants, buffers, bacteriostatic agents or isotonizing agents. Aqueous and nonaqueous aseptic suspensions may also be used, and may include suspending agents, solubilizing agents, thickeners, stabilizers or antiseptic agents. The formulation may be encapsulated in a unit dosage such as an ampule or vial, or in a multiple-dose container. The active ingredient and the pharmacologically acceptable carrier may be lyophilized and stored in a form that can be dissolved or suspended in an appropriate aseptic vehicle just prior to use. Another type of formulation suitable for parenteral administration is a spray formulation.

The content of the complex of the invention in the pharmaceutical composition may be, for example, about 0.1 to 100 wt% of the entire pharmaceutical composition.

The dose of the pharmaceutical composition of the invention will differ depending on the purpose of administration, the method of administration, the type of target disease, the severity and the condition of the administered individual (such as gender, age and body weight), but in the case of systemic administration to an adult, for example, it is usually preferred to be 0.01 mg/kg to 1000 mg/kg, as a single dose of the complex of the invention, or for local administration it is preferred to be from 0.001 mg/body to 100 mg/body. The dose is preferably given 1 to 10 times, and more preferably 5 to 10 times.

The pharmaceutical composition of the invention, for example, may also be used in combination with an existing commercial therapeutic agent for a disease. Such a concomitant drug may be formulated together with the pharmaceutical composition of the invention and administered as a single formulation, or it may be formulated separately from the pharmaceutical composition of the invention and administered either by the same or a different route from the pharmaceutical composition of the invention, and either simultaneously or at a different time. The dose of such other concomitant drugs may be doses normally used when the drugs are administered alone, or they may be reduced below the normally used doses.

### 3. Method for designing antisense oligonucleotide complex with lowered toxicity

As mentioned above, the complex of the invention having the aforementioned structure can exhibit an antisense effect for target RNA while having low toxicity. It is therefore possible to design double-strand nucleic acid having reduced toxicity (in other words, lower toxicity) than conventional single-stranded antisense oligonucleotide or DNA/RNA heteroduplex oligonucleotide (HDO) which have toxicity. Specifically, the invention provides a method for designing a double-strand nucleic acid with reduced toxicity, the method comprising:
(1) a step of selecting an antisense oligonucleotide, and
(2) a step of designing a complementary strand in which the antisense oligonucleotide has a toehold with a single-stranded structure when a double-strand has been formed with the antisense oligonucleotide, and wherein
the complementary strand has nuclease resistance across the full length (this method may hereunder also be referred to as "design method of the invention").

In step (2) of the design method of the invention, the antisense oligonucleotide may have a toehold with a single-stranded structure after formation of the double-strand structure, by modification of the antisense oligonucleotide (for example, insertion or addition of a nucleotide) selected in step (1).

The antisense oligonucleotide selected in step (1) of the design method of the invention may be an antisense oligonucleotide composing a double-strand nucleic acid in which the complementary strand is a complementary strand without resistance to nucleases such as RNase H (for example, a complementary strand comprising a region with 4 or more consecutive natural nucleotides) (this may also be referred to hereunder as "HDO"). In such cases the complementary strand and/or antisense oligonucleotide forming the HDO may be modified to lower toxicity of the antisense oligonucleotide. According to another aspect of the design method of the invention, a method for designing an antisense oligonucleotide complex with reduced toxicity is provided, the method comprising:
(I) a step of selecting double-strand nucleic acid having an antisense oligonucleotide and a complementary strand comprising a sequence complementary to the antisense oligonucleotide and serving as substrate for a nuclease, and
(II) a step of modifying the complementary strand so that:
   the antisense oligonucleotide has a toehold with a single-stranded structure, and
   the nuclease resistance is exhibited across the full length.

The toehold of the single-stranded structure in step (2) or (II) of the design method of the invention may be present at the 5'-end or the 3'-end of the antisense oligonucleotide, or it may be present at the center section or both ends. It may also be a toehold formed by metabolism of part of the complementary strand in the body. The length of each toehold may be 1 base in length or longer, or at least 1 base in length, at least 2 bases in length, at least 3 bases in length, at least 4 bases in length or at least 5 bases in length. The upper limit for the length of the toehold is not particularly restricted but may be 15 bases in length or less, 14 bases in length or less, 13 bases in length or less, 12 bases in length or less, 11 bases in length or less, 10 bases in length or less, 9 bases in length or less, 8 bases in length or less, 7 bases in length or less or 6 bases in length or less. The length of the toehold may be 1-15 bases in length, 1-10 bases in length, 1-7 bases in length, 1-6 bases in length, 3-5 bases in length or 4-5 bases in length.

The complementary strand to be designed or modified in step (2) or (II) of the design method of the invention, after having formed a double-strand with the antisense oligonucleotide, may further have the following features:
(2-1) the double-stranded nucleic acid melting temperature (Tm) is within a specific range, and/or
(2-2) the length is a specific ratio with respect to the length of the antisense strand.

In the design method of the invention, for each nucleic acid strand and its length, the complementary strand, the definition and type of the non-natural oligonucleotide, the measuring method and specific range for the Tm, the method of calculating the length of the complementary strand and the specific ratio with respect to the length of the antisense oligonucleotide, the contents described above under "1. Antisense oligonucleotide complex" and "2. Use of complex of the invention" are incorporated.

The antisense oligonucleotide selected in step (1) or (I) of the design method of the invention may be any publicly known nucleic acid, or it may be newly designed based on sequence information for the target RNA. The antisense oligonucleotide may be a suppression type antisense oligonucleotide or enhancement type antisense oligonucleotide, specific examples of such antisense oligonucleotides including those mentioned above under "1. Antisense oligonucleotide complex".

In step (2) or (II) of the design method of the invention, it is not necessary to actually synthesize the complementary strand, as imagining it is sufficient. Typically, however, the imagined complementary strand will be realized in a program that runs on an electronic computer (for example, oligonucleotide designing software, graphic design tools or office software), or on paper.

The term "modification" for the complementary strand includes the concept of a step of replacing one or more (such as 1, 2, 3, 4, 5 or more) nucleotides composing the complementary strand with another nucleotide, deleting one or more (such as 1, 2, 3, 4, 5 or more) nucleotides composing the complementary strand, inserting or adding one or more (such as 1, 2, 3, 4, 5 or more) nucleotides, as well as replacing one or more (such as 1, 2, 3, 4, 5 or more) nucleotides composing the complementary strand with modified nucleotides or modifying the nucleotides or their internucleoside bonds.

In order for the complementary strand to not be a substrate to nucleases, at least one nucleotide composing the complementary strand may be a non-natural nucleotide, at least one bond between nucleosides of the complementary strand may be a non-phosphodiester bond, a non-natural molecule may be bonded as a cap at the end of the complementary strand, or a combination of these may be employed as appropriate. Such a combination can ensure that the complementary strand does not comprise a region with 4 or more consecutive natural nucleotides. According to one aspect of the invention, it is preferred to replace all of the nucleotides composing the complementary strand with non-natural nucleotides (or to modify the nucleotides), or to replace all of the internucleoside bonds of the complementary strand with modified internucleoside bonds (or to modify the phosphodiester bonds). Examples of modified internucleoside bonds include, but are not limited to, phosphorothioate bonds, phosphorodithioate bonds, phosphotriester bonds, methyl phosphonate bonds, methylthiophosphonate bonds, boranophosphate bonds, phosphoroamidate bonds and so on. According to one embodiment, at least one nucleotide of complementary strand to be designed is a PNA nucleotide, and more preferably all of the nucleotides of the complementary strand are PNA nucleotides.

Each nucleic acid of the antisense oligonucleotide complex designed by the invention may be bonded with one or more (such as 1, 2, 3, 4 or more) functional molecules. For specific examples, bonding forms and bonding methods for such functional molecules, etc., the contents described above under "1. Antisense oligonucleotide complex". are incorporated.

Double-strand nucleic acids with reduced toxicity can be theoretically designed by carrying out the design method of the invention. However, actual lowering of toxicity of the designed double-strand nucleic acid, and the extent of lowering may be evaluated for confirmation.

### 4. Method for producing antisense oligonucleotide complex with reduced toxicity and method for lowering toxicity of antisense oligonucleotide

The present invention provides a method for producing an antisense oligonucleotide complex with reduced toxicity, the method comprising:
(1) a step of preparing an antisense oligonucleotide,
(2) a step of preparing a complementary strand in which the antisense oligonucleotide has a toehold with a single-stranded structure when a double-strand has been formed with the antisense oligonucleotide, and wherein
   the complementary strand has nuclease resistance across the full length, and
(3) a step of annealing the antisense strand prepared in step (1) and the complementary strand prepared in step (2)
(this method will hereunder also be referred to as "production method of the invention").

According to another aspect, the invention provides a method for lowering toxicity of an antisense oligonucleotide, the method comprising:
(I) a step of preparing an antisense oligonucleotide,
(II) a step of preparing a complementary strand in which the antisense oligonucleotide has a toehold with a single-stranded structure when a double-strand has been formed with the antisense oligonucleotide, and wherein
   the complementary strand has nuclease resistance across the full length, and
(III) a step of annealing the antisense oligonucleotide prepared in step (I) and the complementary strand prepared in step (II)
(this method will hereunder also be referred to as "toxicity lowering method of the invention"). The term "method of the invention" will hereunder be used to collectively refer to the production method and toxicity lowering method of the invention.

The toehold of the single-stranded structure in step (2) or (II) of the method of the invention may be present at the 5'-end or the 3'-end of the antisense oligonucleotide, or it may be present at the center section or both ends. It may also be a toehold formed by metabolism of part of the complementary strand in the body. The length of each toehold may be 1 base in length or longer, or at least 1 base in length, at least 2 bases in length, at least 3 bases in length, at least 4 bases in length or at least 5 bases in length. The upper limit for the length of the toehold is not particularly restricted but may be 15 bases in length or less, 14 bases in length or less, 13 bases in length or less, 12 bases in length or less, 11 bases in length or less, 10 bases in length or less, 9 bases in length or less, 8 bases in length or less, 7 bases in length or less or 6 bases in length or less. The length of the toehold may be 1-15 bases in length, 1-10 bases in length, 1-7 bases in length, 1-6 bases in length, 3-5 bases in length or 4-5 bases in length.

The complementary strand prepared in step (2) or (II) of the method of the invention, after having formed a double-strand with the antisense oligonucleotide, may further have the following features:
(2-1) the double-stranded nucleic acid melting temperature (Tm) is within a specific range, and/or
(2-2) the length is a specific ratio with respect to the length of the antisense strand.

For the production method of the invention, each nucleic acid strand and its length, the complementary strand, the definition and type of the non-natural oligonucleotide, the measuring method and specific range for the Tm, the method of calculating the length of the complementary strand, the specific ratio with respect to the length of the antisense oligonucleotide, and the specific method and aspect for each step, etc., the contents described above under "1. Antisense oligonucleotide complex" to "3. Method for designing antisense oligonucleotide complex with lowered toxicity" are incorporated.

The antisense oligonucleotide prepared in step (1) or (I) of the method of the invention may be any publicly known antisense oligonucleotide, or it may be newly designed based on sequence information for the target RNA. The antisense oligonucleotide may be one with known or unknown toxicity (such as hepatotoxicity). The antisense oligonucleotide may be a suppression type antisense oligonucleotide or enhancement type antisense oligonucleotide, specific examples of such antisense oligonucleotides including those mentioned above under "1. Antisense oligonucleotide complex". The complementary strand prepared in step (2) or (II) of the method of the invention may also be a complementary strand designed by the design method of the invention. The nucleic acids may be synthesized by a publicly known method, or they may be purchased as commercial products or obtained by ordering from a manufacturer. The method may therefore include, prior to step (2) or (II) of the method of the invention, a step of designing the antisense oligonucleotide complex by the design method of the invention and/or a step of synthesizing each nucleic acid for the complex.

The annealing in step (3) or (III) of the method of the invention may be carried out, for example, by mixing prepared nucleic acid in a suitable buffer solution, denaturing for several minutes (such as 5 minutes) at about 90°C to 98°C, and then treating the nucleic acid for about 1 to 8 hours at about 30°C to 70°C.

The antisense oligonucleotide and/or complementary strand prepared in step (1) or (I) of the method of the invention may be bonded with one or more (such as 1, 2, 3, 4 or more) functional molecules in advance, or the antisense oligonucleotide and/or complementary strand may be bonded with a functional molecule at an arbitrary timing in the method of the invention. For specific examples, bonding forms and bonding methods for such functional molecules, etc., the contents described above under "1. Antisense oligonucleotide complex" are incorporated.

As used herein, the term "reduced toxicity" means that the toxicity exhibited when a nucleic acid of interest (the nucleic acid selected in step (I)) is administered to cells or an organism is reduced or eliminated when the antisense oligonucleotide complex processed by the toxicity lowering method of the invention has been administered under the same conditions.

A composition with reduced toxicity can also be produced by using a publicly known method to formulate double-strand nucleic acid with reduced toxicity produced by the production method of the invention.

The actual lowering of toxicity of the antisense oligonucleotide complex produced by the method of the invention, and the extent of lowering, may be evaluated for confirmation.

The invention will now be described in greater detail by Examples, with the understanding that the invention is not limited thereto in any way.

### EXAMPLES

### [Example 1]

An *in vivo* experiment was conducted to verify *in vivo* activity and alleviating effects on hepatotoxicity and nephrotoxicity, with subcutaneous administration of double-strand nucleic acid agents.

In this Example, the double-strand nucleic acid agents listed in Table 1 having double-strand nucleic acid forms were evaluated for *in vivo* activity and alleviation of hepatotoxicity and nephrotoxicity, using the single-stranded LNA/DNA gapmer antisense oligonucleotides (hereunder referred to as "ASO") (mPCS 1) listed in Table 1 as a control. In the Sequence Listing, SEQ ID NO: 1 to 6 are the sequences in Table 1 in order from the top (excluding apPNA(C8)-mPCS 1 and apPNA(C8)G2-mPCS 1, which are less than 10 bases in length). The sequences listed as SEQ ID NO: 1 and 2 are antisense strand sequences, and the sequences listed as SEQ ID NO: 3 to 6 are complementary strand sequences.

**[Table 1]**

| | | -3 | -2 | -1 | 1 | 2 | 3 | 4 | 5 | 6 | 7 | 8 | 9 | 10 | 11 | 12 | 13 | 14 | |
|---|---|---|---|---|---|---|---|---|---|---|---|---|---|---|---|---|---|---|---|
| Abbreviation | ID | Sequence | | | | | | | | | | | | | | | | | |
| mPCS1 | mPCSK9-2590-BNA(14) | 5' | Y | Y | A | C | A | c | c | a | a | g | t | t | c | T | C | c | 3' |
| mPCSlNoG | NoGal-mPCSK9-2590-BNA(14) | | | | A | C | A | c | c | a | a | g | t | t | c | T | C | c | |
| apPNA(12)-mPCS1 | apPNA(12)-mPCSK9-2590-BNA(14) | | | | | C-term | T | G | G | T | T | C | A | A | G | A | G | G | N-term |
| apPNA(11)-mPCS1 | apPNA(11)-mPCSK9-2590-BNA(14) | | | | | | C-term | G | G | T | T | C | A | A | G | A | G | G | N-term |
| apPNA(10)-mPCS1 | apPNA(10)-mPCSK9-2590-BNA(14) | | | | | | | C-term | G | T | T | C | A | A | G | A | G | G | N-term |
| apPNA(C8)-mPCS1 | apPNA(C8)-mPCSK9-2590-BNA(14) | | | C-term | T | G | T | G | G | T | T | C | N-term | | | | | | |
| apPNA(C8)G2-mPCS1 | apPNA(Gal2C8)-mPCSK9-2590-BNA(14) | C-term | Z | Z | T | G | T | G | G | T | T | C | N-term | | | | | | |
| cRNA(14)-mPCS1 | cRNA(r14)-mPCSK9-2590-BNA(14) | | | 3' | rU | rG | rU | rG | rG | rU | rU | rC | rA | rA | rG | rA | rG | rG | 5' |

| | | | | | | | | | | | | | | | | | | | |
|---|---|---|---|---|---|---|---|---|---|---|---|---|---|---|---|---|---|---|---|
| N: LNA, n: DNA, Y: GalNAc_{apd} N: PNA, rN: RNA, Z: GalNAc for PNA | | | | | | | | | | | | | | | | | | | |

### (Nucleic acid synthesis)

The listed oligonucleotides were synthesized on a DNA/RNA synthesizer (NTS M-2-TRS, Nihon Techno Service Co., Ltd.). All phosphoramidite monomers (dA (Bz), dG (iBu), dC (Bz), dC (Bz), T, LNA-A (Bz), LNA-G (DMF), LNA-mC (Bz) and LNA-T) were dissolved in anhydrous acetonitrile to a final concentration of 0.067 M. The oligonucleotides were synthesized at a 1.0 µmol scale using standard solid-phase oligonucleotide synthesis with 5-ethylthio-1*H*-tetrazole (0.25 M anhydrous acetonitrile) as an activator. The coupling time was 6 minutes for LNA phosphoramidites and 30 seconds for natural phosphoramidites. After synthesis, the solid-phase support was transferred to a 1.0 mL gas-tight syringe and the GalNAc ligand was extended using standard solid-phase oligonucleotide synthesis manually with 5-ethylthio-1*H*-tetrazole (0.25 M anhydrous acetonitrile). 5'-DMTr-protected oligonucleotides were treated with 28% aquenous ammonia at 55°C for 13 hours cleaved from solid-phase support and base and cyanoethyl protecting groups were removed. The crude oligonucleotides were purified using Glen-Pak^{™} DNA Purification Cartridge (Glen Research, Inc.). The resulting oligonucleotides were further purified by reverse-phase HPLC. The HPLC conditions were as follows.

Eluent solution A: 100 mM hexafluoro-2-propanol, 8.6 mM triethylamine (pH 8.3)
Solution B: methanol
Gradient solution B concentration: 5-30% (30 min) (purification)
   5-40% (20 min) (purity confirmation)
Columns: Nacalai ⁵C18-MS-II (10 × 250 mm) (purification)
   Nacalai ⁵C18-MS-II (4.6 × 50 mm) (purity confirmation)
Flow rates: 1.5 mL/min (purification)
   0.5 mL/min (purity confirmation)
Column temperature: 60°C
Detection: UV (260 nm)

### (GalNAc phosphoramidite synthesis)

Two types of *N*-acetylgalactosamine (GalNAc) phosphoramidites were synthesized using the methods described in the following non-patent literature.

### References

1. Curr Protoc Nucleic Acid Chem. 2019,78, e99. Doi: 10.1002/cpnc.99.
2. Nucleic Acid Ther. 2021, in press. Doi: 10.1089/nat.2021.0036.

### (Synthesis of GalNAc monomer for PNA synthesis)

Fmoc-Orn-OBn and GalNAc carboxylic acid (see Reference 1) were condensed, followed by deprotection of the benzyl group was deprotected, to obtain the following compound Fmoc-Om(GalNAc)-OH (Chemical Formula 7).

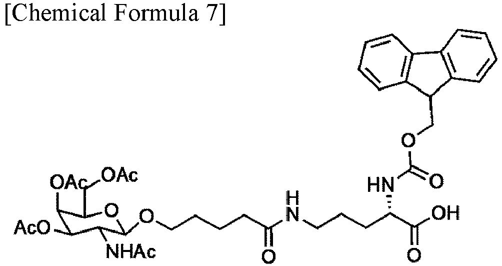

The PNA oligomers listed in Table 1 were synthesized using commercially available Fmoc-A(Bhoc)-aeg-OH, Fmoc-G(Bhoc)-aeg-OH, Fmoc-C(Bhoc)-aeg-OH, Fmoc-T-aeg-OH and separately synthesized Fmoc-Om(GalNAc)-OH (Chemical Formula 7) according to References 3 and 4.

### References

3. Tetrahedron Letters, 2010, 51 3716-3718
4. J. Org. Chem. 2020, 85, 14,8812-8824

### (Melting temperature (Tₘ) measurement (evaluation of duplex forming ability))

The oligonucleotide solution was prepared to a final concentration of 1 µM (mPCS1/apPNA(12)), 2 µM (mPCS1NoG/apPNA(C8)G2, mPCS1/cRNA(14)) or 4 µM in a 10 mM sodium phosphate buffer (pH 7.0) containing 100 mM NaCl and 0.1 mM ethylenediaminetetraacetic acid. The mixture was annealed by heating at 95°C for 3 minutes and then cooled at a rate of 1°C per minute to 20°C. The melting temperature (*T*ₘ) was estimated using the software provided with the TMSPC-8, defined as the temperature at which the formed duplexes were half-dissociated. The temperature-dependent change in optical absorption was recorded at 260 nm, with a scan rate of 0.5°C per minute from 4°C to 90°C. Each averaged *T*ₘ value was determined as the intersection point between the absorption versus temperature curves.

### (KD activity measurement of ASO in mouse liver)

All animal experiments were conducted with the approval of the Animal Experimentation Committee of Nagasaki University (Nagasaki, Japan). Equimolar mixtures of ASO and PNA were annealed by overnight immersion in hot water followed by gradual cooling. Male mice (7-week-old C57BL/6J) were purchased from SLC Japan (Tokyo, Japan). ASO or annealed double-strand nucleic acids were subcutaneously administered to the mice at dose of 17.5 nmol/kg. After 72 hours of a single administration, the mice were anesthetized with isoflurane (Pfizer Japan, Tokyo, Japan) and euthanized to collect their livers and kidneys. Liver tissues were stored overnight in RNAlater^{™} at 4°C and then kept at -20°C until analysis. Total RNA was extracted using a QuickGene RNA tissue kit SII (FujiFilm Corp.) according to the accompanying manual. Reverse transcription was carried out using a High-Capacity RNA-to-cDNA^{™} kit (Thermo Fisher Scientific) with the extracted total RNA as template, and then the expression level of PCSK9 mRNA was analyzed using a CFX real-time PCR system (Bio-Rad). The primer sets used were mPCSK9-F: 5'-TCAGTTCTGCACACCTCCAG-3' (SEQ ID NO: 21) and mPCSK9-R: 5'-GGGTAAGGTGCGGTAAGTCC-3' (SEQ ID NO: 22) for analysis of mouse PCSK9, and mGAPDH-F: 5'-TGTGTCCGTCGTGGATCTGA-3' (SEQ ID NO: 23) and mGAPDH-R: 5'-TTGCTGTTGAAGTCGCAGGAG-3' (SEQ ID NO: 24) for analysis of mouse Gapdh as a housekeeping gene. The KD activity was calculated based on the relative expression level in terms of the difference in Ct value as the difference in expression level.

### (Blood biochemistry test in mice)

Peripheral blood was collected in BD Microtainer tubes (BD, Franklin Lakes, NJ) to separate the serum. The serum AST/ALT and CRE were measured using Fuji dry-chem slide. An aliquot of 10 µL of serum was used for each measurement.

The results are shown in Fig. 2. Fig. 2 shows that liver toxicity (increased AST and ALT) and kidney toxicity (increased CRE) were reduced by using PNA as the complementary strand in combination with single-stranded ASO which exhibits high hepatotoxicity. When the liver-targeting ligand GalNAc (Chemical Formula 7) was introduced into full PNA having resistance to different nucleases across the full length of the complementary strand, as the second strand, activity in the liver was significantly attenuated, suggesting that in order to effectively obtain the ligand activity, the complementary strand must be cut by nucleases releasing ASO from the complementary strand at an appropriate timing as indicated by HDO.

The present inventors have previously developed a method for effectively introducing a targeting ligand into ASO, and by combining a complementary strand such as apPNA(C8)-mPCS1 with the ASO (mPCS1), succeeded in lowering hepatotoxicity and nephrotoxicity while retaining antisense activity. When a complementary strand without resistance to nucleases such as RNase H (cRNA (14)-mPCS1) was used for mPCS1, no loss of toxicity was achieved.

Fig. 2 shows that with a double-strand nucleic acid having GalNAc on the complementary strand side and without GalNAc on the ASO side, the antisense effect was greatly attenuated, raising the suspicion that the double-strand nucleic acid had not migrated to the liver. Migration of the double-strand nucleic acid into the liver was therefore examined.

6-week-old male BALB/c nu-nu mice used in this study were purchased from SLC Japan (Tokyo, Japan). Mice were fed an autofluorescence-reduced diet (D10001, Research Diets, Inc.) for one week before assays were commenced. Alexa Fluor 647-labeled oligonucleotides were dissolved in saline for injection (300 pmol in 200 µL saline). Mice were intravenously injected with a single dose of either mPCS 1NoGaf or BROTHERS complex (mPCS1NoGaf/apPNA(C8)G2) via tail vein. The biodistribution was visualized using an IVIS Lumina II (Caliper Life Science) at different time points. The sequences of the nucleic acids used are shown in Table 2 and SEQ ID NO: 7.

**Table 2]**

| | | -3 | -2 | -1 | 1 | 2 | 3 | 4 | 5 | 6 | 7 | 8 | 9 | 10 | 11 | 12 | 13 | 14 | |
|---|---|---|---|---|---|---|---|---|---|---|---|---|---|---|---|---|---|---|---|
| Abbreviation | ID | Sequence | | | | | | | | | | | | | | | | | |
| mPCSINoGaf | NoGal-mPCSK9-2590-BNA(14) | | | | A | C | A | c | c | a | a | g | t | t | c | T | C | c | L |
| apPNA(C8)G2 | apPNA(Gal2C8)-mPCSK9-2590-BNA(14) | C-term | Z | Z | T | G | T | G | G | T | T | C | N-term | | | | | | |

| | | | | | | | | | | | | | | | | | | | |
|---|---|---|---|---|---|---|---|---|---|---|---|---|---|---|---|---|---|---|---|
| N: LNA, n: DNA, L: AlexaFluor647 N: PNA, rN: RNA, Z: GalNAc for PNA | | | | | | | | | | | | | | | | | | | |

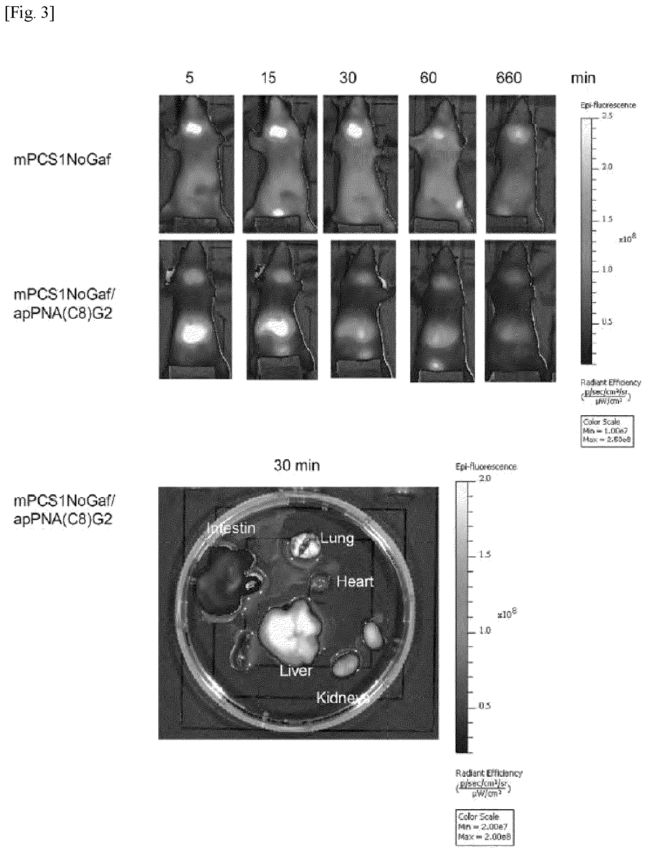

The results are shown in Fig. 3. Fig. 3 shows that even the double-strand nucleic acid having GalNAc on the complementary strand side without GalNAc on the ASO side had migrated to the liver. This suggested that the BROTHERS method with a ligand on the ASO side exhibits an antisense effect by a different mechanism from HDO having a ligand on the complementary strand.

### [Example 2]

Bound proteins were visualized to verify whether BROTHERS technology can attenuate protein interaction, which is known as a hybridization-independent off-target effect.

Mouse liver slices were homogenized and centrifuged for 10 minutes at 10,000 g, and the supernatant was collected to prepare liver lysate. The lysate was diluted with a 100 mM potassium chloride solution to a concentration of 3.0 mg/mL. Streptavidin magnetic beads (500 µg) were then dispersed in PBS and magnetically separated to remove the supernatant. The beads were washed three times in PBS, and then incubated with biotin-labeled single-stranded ASO (mPCS1B) and BROTHERS complex (mPCS1B/apPNA(C8)) (100 µM, 50 µL in PBS) at 4°C for 1 h. Beads without immobilized nucleic acid were used as a negative control (NT). After magnetic separation, the beads were washed 3 times. The beads were dispersed in PBS (200 µL) and then dispensed separately into two tubes. After magnetic separation and removal of the supernatant, complementary strand nucleic acid (125 µM, 20 µL) was added to one of the tubes, while PBS (20 µL) was added to the other tube. The beads were incubated at 4°C for 2 h with mouse liver lysate (3.0 mg/mL, 150 µL). After magnetic separation, the supernatant was removed and the beads were washed three times. The beads were incubated with 500 pmol competitor non-biotylated nucleic acids (mPCS1NoG and mPCS1NoG/apPNA(C8)) for 5 minutes at room temperature. After magnetic separation, supernatants were collected as eluted proteins and the beads were incubated with competitor non-biotylated nucleic acids again. After magnetic separation, supernatants were collected. The eluted proteins were visualized by SDS-PAGE according to an established method. Specifically, a marker and each sample were applied to a ^{m}PAGE^{™} 4% to 20% Bis-Tris gel and electrophoresis was carried out for 40 minutes at 180 V. After CBB staining for 60 minutes, it was decolorized for 90 minutes with CBB decolorizing solution, and then replaced with ultrapure water and shaken overnight. A CBB stained gel image was obtained using a ChemiDoc^{™} Touch imaging system (Bio-Rad) (N=3). The sequence of the mPCS1B is shown in Table 3 and SEQ ID NO: 8.

**[Table 3]**

| | | -3 | -2 | -1 | 1 | 2 | 3 | 4 | 5 | 6 | 7 | 8 | 9 | 10 | 11 | 12 | 13 | 14 | |
|---|---|---|---|---|---|---|---|---|---|---|---|---|---|---|---|---|---|---|---|
| Abbreviation | ID | Sequence | | | | | | | | | | | | | | | | | |
| mPCS1B | mPCSK9-2590-BNA(14) -biotin | | 5' | b | A | C | A | c | c | a | a | g | t | t | c | T | C | c | 3' |

| | | | | | | | | | | | | | | | | | | | |
|---|---|---|---|---|---|---|---|---|---|---|---|---|---|---|---|---|---|---|---|
| N: LNA, n: DNA, b: biotin | | | | | | | | | | | | | | | | | | | |

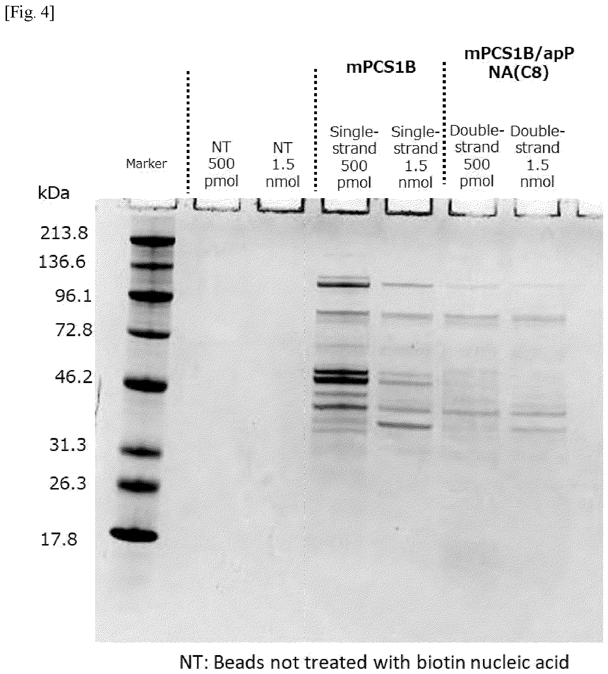

Fig. 4 shows an example of a CBB stained image of gel. With single-stranded ASO which exhibits strong toxicity, binding of proteins of many types and amounts was observed. With the BROTHERS complex (mPCS 1 NoG/apPNA(C 8)), on the other hand, a clear reduction in binding amount and decrease in types of bound proteins was confirmed.

This proved that BROTHERS technology can reduce the amount of bound protein or reduce the types of bound proteins, making it possible to reduce hybridization-independent off-target binding. This suggests significant lowering of toxicity as a nucleic acid drug.

### [Example 3]

In this Example, the double-stranded agents listed in Table 4 having double-strand nucleic acid forms were evaluated for *in vivo* activity and alleviation of hepatotoxicity and nephrotoxicity, using the single-stranded LNA/DNA gapmer antisense oligonucleotides (hereunder referred to as "ASO") (GN6) listed in Table 4 as controls. In the Sequence Listing, SEQ ID NO: 9 to 13 are the sequences in Table 4 in order from the top (excluding apPNA(8)-GN6 and apPNA(C8)-GN6 which were less than 10 bases in length). The sequence listed as SEQ ID NO: 9 is the antisense strand sequence, and the sequences listed as SEQ ID NO: 10 to 14 are complementary strand sequences.

**Table 4]**

| | | -3 | -2 | -1 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|---|---|
| Abbreviation | ID | Sequence | | | | | | | | | | | | | | | | |
| GN6 | hApoB-10177-BNA(13) | X | X | X | G | C | a | t | t | g | g | t | a | t | T | C | A | 3' |
| apPNA(12)-GN6 | | | | | C-term | G | T | A | A | C | C | A | T | A | A | G | T | N-term |
| apPNA(11)-GN6 | | | | | | C-term | T | A | A | C | C | A | T | A | A | G | T | N-term |
| apPNA(10)-GN6 | | | | | | | C-term | A | A | C | C | A | T | A | A | G | T | N-term |
| apPNA(9)-GN6 | | | | | | | | C-term | A | C | C | A | T | A | A | G | T | N-term |
| apPNA(8)-GN6 | | | | | | | | | C-term | C | C | A | T | A | A | G | T | N-term |
| apPNA(C8)-GN6 | | | | C-term | C | G | T | A | A | C | C | A | N-term | | | | | |
| cRNA-GN6 | | | | 3' | rC | rG | rU | rA | rA | rC | rC | rA | rU | rA | rA | rG | rU | 5' |

| | | | | | | | | | | | | | | | | | | |
|---|---|---|---|---|---|---|---|---|---|---|---|---|---|---|---|---|---|---|
| N: LNA, n: DNA, X: GalNAcₕₚ, N: PNA, rN: RNA | | | | | | | | | | | | | | | | | | |

### (Nucleic acid synthesis)

The antisense oligonucleotide was synthesized by GeneDesign, Inc. (Osaka, Japan).

### (Synthesis of PNA)

The PNA oligomers listed in Table 4 were synthesized using commercially available Fmoc-A (Bhoc)-aeg-OH, Fmoc-G (Bhoc)-aeg-OH, Fmoc-C (Bhoc)-aeg-OH, Fmoc-T-aeg-OH and separately synthesized Fmoc-Om(GalNAc)-OH (Chemical Formula 7) according to References 3 and 4 mentioned above.

### (Melting temperature (Tm) measurement (evaluation of double-strand forming ability))

The oligonucleotide solution was prepared to a final concentration of 1 µM or 4 µM in a 10 mM sodium phosphate buffer (pH 7.0) containing 100 mM NaCl and 0.1 mM ethylenediaminetetraacetic acid. The mixture was annealed by heating at 95°C for 3 minutes and then cooled at a rate of 1°C per minute to 20°C. The melting temperature (*T*ₘ) was estimated using the software provided with the TMSPC-8, defined as the temperature at which the formed duplexes were half-dissociated. The temperature-dependent change in optical absorption was recorded at 260 nm, with a scan rate of 0.5°C per minute from 4°C to 90°C. Each averaged *T*ₘ value was determined as the intersection point between the absorption versus temperature curves.

### (KD activity measurement of ASO in mouse liver)

All animal experiments were conducted with the approval of the Animal Experimentation Committee of Nagasaki University (Nagasaki, Japan). Equimolar mixtures of ASO and PNA were annealed by overnight immersion in hot water followed by gradual cooling. Male mice (7-week-old C57BL/6J) were purchased from SLC Japan (Tokyo, Japan). ASO or annealed double-strand nucleic acids were subcutaneously administered to the mice at dose of 100 nmol/kg. After 72 hours of a single administration, the mice were anesthetized with isoflurane (Pfizer Japan, Tokyo, Japan) and euthanized to collect their livers and kidneys. Liver tissues were stored overnight in RNAlater^{™} at 4°C and then kept at -20°C until analysis. Total RNA of the livers was extracted using a QuickGene RNA tissue kit SII (FujiFilm Corp.) according to the accompanying manual. Reverse transcription was carried out using a High-Capacity RNA-to-cDNA^{™} kit (Thermo Fisher Scientific) with the extracted total RNA as template, and then the expression level of ApoB mRNA was analyzed using a CFX real-time PCR system (Bio-Rad). The primer sets used were mApoB-F: 5'-TCCTCGGTGAGTTCAATGACTTTC-3' (SEQ ID NO: 25) and mApoB-R: 5'-TGGACCTGCTGTAGCTTGTAGGA-3' (SEQ ID NO: 26) for analysis of mouse ApoB, and mGAPDH-F: 5'-TGTGTCCGTCGTGGATCTGA-3' and mGAPDH-R: 5'-TTGCTGTTGAAGTCGCAGGAG-3' for analysis of mouse Gapdh as a housekeeping gene, and the KD activity was calculated based on the relative expression level in terms of the difference in Ct value as the difference in expression level. The off-target effect was evaluated by selecting out several genes containing 1 bp mismatch and using the following primers for analysis of the mRNA expression level by the same method described above. Plastin1-Fw: gtg aag ccg aag atg gtg at (SEQ ID NO: 27), Plastin1-Rv:gtt tgg tgc gta cag tgt gg (SEQ ID NO: 28), Lrp2-Fw: ggt tcg tta tgg cag tcg tt (SEQ ID NO: 29), Lrp2-Rv: tgc tgg ctt gga aga ctt tt (SEQ ID NO: 30), Atp5c1-Fw: tga age cct gtg aaa cac tg (SEQ ID NO: 31), Atp5c1-Rv: agc aaa gag tcg gat ggc ta (SEQ ID NO: 32), Pirin-Fw: cat ttc agg aga age ctt gg (SEQ ID NO: 33), Pirin-Rv:agg aat agg ctg gga atg ct (SEQ ID NO: 34), Dpyd-Fw: etc gca aaa gcc tat tcc tg (SEQ ID NO: 35), Dpyd-Rv: atc agg gcc aca act tgt tc (SEQ ID NO: 36)

### (Blood biochemistry test in mice)

Peripheral blood was collected in BD Microtainer tubes (BD, Franklin Lakes, NJ) to separate the serum. The serum ALT and CRE were measured using Fuji dry-chem slide. An aliquot of 10 µL of serum was used for each measurement.

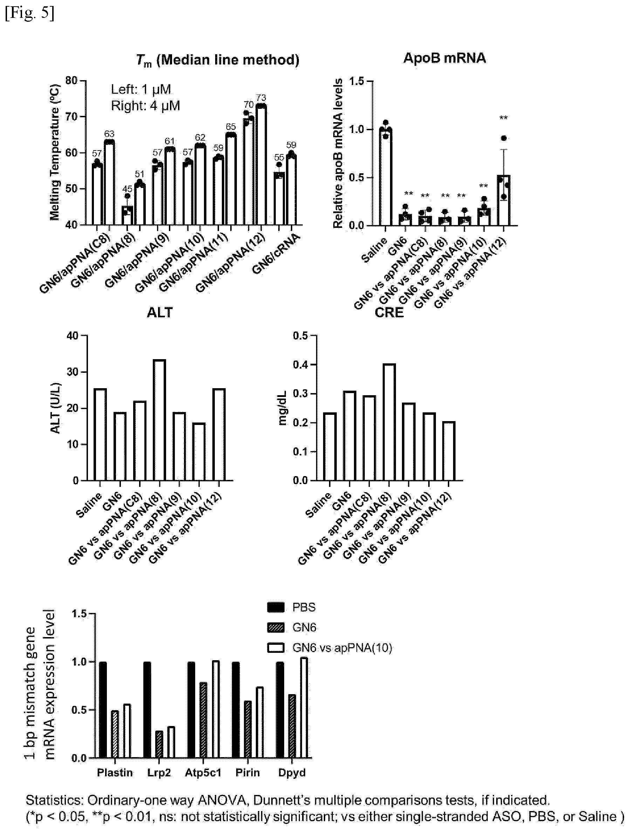

The results are shown in Fig. 5. Fig. 5 shows that even with sequences different from the previous Example 1, using PNA as the complementary strand for single-stranded ASO changes knockdown (KD) activity of the gene depending on the thermodynamic stability of the double-strand and the length of the single-stranded portion. The antisense effect was found to be lower with increasing double-strand binding strength, under conditions without fluctuation in toxicity markers. GN6/apPNA(10)-GN6, where the expected BROTHERS phenomenon seemed to have occurred most notably, was analyzed for mRNA expression level of several selected genes containing mismatches in the target sequence, in order to evaluate how the off-target effect was influenced. The results showed knockdown as an off-target effect with the single-stranded GN6, in contrast to elimination or attenuation of off-target knockdown of the examined genes when a BROTHERS complex was used (Fig. 5). These results indicated that a BROTHERS double-strand nucleic acid has a property that avoids the off-target effect.

### [Example 4]

In this Example, the double-stranded agents listed in Table 4 having double-strand nucleic acid forms were evaluated for *in vivo* activity and alleviation of hepatotoxicity and nephrotoxicity, using the single-stranded LNA/DNA gapmer antisense oligonucleotides (hereunder referred to as "ASO") (hApo1) listed in Table 4 as controls. In the Sequence Listing, SEQ ID NO: 15 to 20 are the sequences in Table 4 in order from the top (excluding apPNA(C5)-hApo1, apPNA(C6)-hApo1, apPNA(C7)-hApo1, apPNA(C8)-hApo1 and apPNA(C9)-hApo1 which were less than 10 bases in length). The sequence listed as SEQ ID NO: 15 is the antisense strand sequence, and the sequences listed as SEQ ID NO: 16 to 20 are complementary strand sequences.

**[Table 5]**

| | | -3 | -2 | -1 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|---|---|
| Abbreviation | ID | Sequence | | | | | | | | | | | | | | | | |
| hApo1 | hApoB-521-BNA(13) | 5' | Y | Y | A | A | t | g | g | c | c | a | g | c | T | T | G | 3' |
| apPNA(C5)-hApo1 | apPNA(C5)-hApoB-521-BNA(13) | | | C-term | T | T | A | C | C | N-term | | | | | | | | |
| apPNA(C6)-hApo1 | apPNA(C6)-hApoB-521-BNA(13) | | | C-term | T | T | A | C | C | G | N-term | | | | | | | |
| apPNA(C7)-hApo1 | apPNA(C7)-hApoB-521-BNA(13) | | | C-term | T | T | A | C | C | G | G | N-term | | | | | | |
| apPNA(C8)-hApo1 | apPNA(C8)-hApoB-521-BNA(13) | | | C-term | T | T | A | C | C | G | G | T | N-term | | | | | |
| apPNA(C9)-hApo1 | apPNA(C9)-hApoB-521-BNA(13) | | | C-term | T | T | A | C | C | G | G | T | C | N-term | | | | |
| apPNA(C10)-hApo1 | apPNA(C10)-hApoB-521-BNA(13) | | | C-term | T | T | A | C | C | G | G | T | C | G | N-term | | | |
| apPNA(C1)-hApo1 | apPNA(C11)-hApoB-521-BNA(13) | | | C-term | T | T | A | C | C | G | G | T | C | G | A | N-term | | |
| apPNA(C12)-hApo1 | apPNA(C12)-hApoB-521-BNA(13) | | | C-term | T | T | A | C | C | G | G | T | C | G | A | A | N-term | |
| apPNA(C13)-hApo1 | apPNA(C13)-hApoB-521-BNA(13) | | | C-term | T | T | A | C | C | G | G | T | C | G | A | A | C | N-term |
| cRNA-hApo1 | cRNA(13)-hApoB-521-BNA(13) | | 3' | | rU | rU | rA | rC | rC | rG | rG | rU | rC | rG | rA | rA | rC | 5' |

| | | | | | | | | | | | | | | | | | | |
|---|---|---|---|---|---|---|---|---|---|---|---|---|---|---|---|---|---|---|
| N: LNA, n: DNA, rN: RNA, Y: GalNAc_{apd}, N: PNA | | | | | | | | | | | | | | | | | | |

### (Nucleic acid synthesis)

The listed oligonucleotides were synthesized on a DNA/RNA synthesizer (NTS M-2-TRS by Nihon Techno Service Co., Ltd.). All phosphoramidite monomers (dA (Bz), dG (iBu), dC (Bz), dC (Bz), T, LNA-A (Bz), LNA-G (DMF), LNA-mC (Bz) and LNA-T) were dissolved in anhydrous acetonitrile to a final concentration of 0.067 M. The oligonucleotides were synthesized at a 1 µmol scale using standard solid-phase oligonucleotide synthesis with 5-benzylthio-1H-tetrazole (0.25 M anhydrous acetonitrile) as an activator. The coupling time was 6 minutes for LNA phosphoramidites and 30 seconds for natural amidite phosphoramidites. After synthesis, the solid-phase support was transferred to a 1.0 mL gas-tight syringe and the GaNAc ligand was extended using standard solid-phase oligonucleotide synthesis manually with 5-ethylthio-1H-tetrazole (0.25 M anhydrous acetonitrile) as an activator. 5'-DMTr-protected oligonucleotides were treated with 28% aquenous ammonia at 55°C for 13 hours cleaved from solid-phase support and base and cyanoethyl protecting groups were removed. The crude oligonucleotides were purified using Glen-Pak^{™} DNA Purification Cartridge (Glen Research, Inc.). The resulting oligonucleotides were futher purified by reverse-phase HPLC. The HPLC conditions were as follows.

Eluent solution A: 100 mM hexafluoro-2-propanol, 8.6 mM triethylamine (pH 8.3)
Solution B: methanol
Gradient solution B concentration: 5-30% (30 min) (purification)
   5-40% (20 min) (purity confirmation)
Columns: Nacalai ⁵C18-MS-II (10 × 250 mm) (purification)
   Nacalai ⁵C18-MS-II (4.6 × 50 mm) (purity confirmation)
Flow rates: 1.5 mL/min (purification)
   0.5 mL/min (purity confirmation)
Column temperature: 60°C
Detection: UV (260 nm)

### (PNA synthesis)

The PNA oligomers listed in Table 5 were synthesized using commercially available Fmoc-A (Bhoc)-aeg-OH, Fmoc-G (Bhoc)-aeg-OH, Fmoc-C (Bhoc)-aeg-OH, Fmoc-T-aeg-OH and separately synthesized Fmoc-Om(GalNAc)-OH (Chemical Formula 7) according to References 3 and 4 mentioned above.

### (Melting temperature (Tₘ) measurement (evaluation of duplex forming ability))

Each oligonucleotide solution was prepared to a final concentration of 4 µM in a 10 mM sodium phosphate buffer (pH 7.0) containing 100 mM NaCl and 0.1 mM ethylenediaminetetraacetic acid. The mixture was annealed by heating at 95°C for 3 minutes and then cooled at a rate of 1°C per minute to 20°C. The melting temperature (Tm) was estimated using the software provided with the TMSPC-8, defined as the temperature at which the formed duplexes were half-dissociated. The temperature-dependent change in optical absorption was recorded at 260 nm, with a scan rate of 0.5°C per minute from 4°C to 90°C. Each averaged *T*ₘ value was determined as the intersection point between the absorption versus temperature curves.

### (KD activity measurement of ASO in mouse liver)

All animal experiments were conducted with the approval of the Animal Experimentation Committee of Nagasaki University (Nagasaki, Japan). Equimolar mixtures of ApoB-targeting ASO and PNA were annealed by overnight immersion in hot water followed by gradual cooling. Male mice (7-week-old C57Bl/6J) were purchased from SLC Japan (Tokyo, Japan). ASO or annealed double-strand nucleic acids were subcutaneously administered to the mice at dose of 100 nmol/kg. After 72 hours of a single administration, the mice were anesthetized with isoflurane (Pfizer Japan, Tokyo, Japan) and euthanized to collect their livers and kidneys. Liver tissues were stored overnight in RNAlater^{™} at 4°C and then kept at -20°C until analysis. Total RNA of the livers was extracted using a QuickGene RNA tissue kit SII (FujiFilm Corp.) according to the accompanying manual. Reverse transcription was carried out using a High-Capacity RNA-to-cDNA^{™} kit (Thermo Fisher Scientific) with the extracted total RNA as template, and then the expression level of ApoB mRNA was analyzed using a CFX real-time PCR system (Bio-Rad). The primer sets used were mApoB-F: 5'-TCCTCGGTGAGTTCAATGACTTTC-3' and mApoB-R: 5'-TGGACCTGCTGTAGCTTGTAGGA-3' for analysis of mouse ApoB, and mGAPDH-F: 5'-TGTGTCCGTCGTGGATCTGA-3' and mGAPDH-R: 5'-TTGCTGTTGAAGTCGCAGGAG-3' for analysis of mouse Gapdh as a housekeeping gene, and the KD activity was calculated based on the relative expression level in terms of the difference in Ct value as the difference in expression level.

### (Blood biochemistry test in mice)

Peripheral blood was collected in BD Microtainer tubes (BD, Franklin Lakes, NJ) to separate the serum. The serum ALT and CRE were measured using Fuji dry-chem slide. An aliquot of 10 µL of serum was used for each measurement.

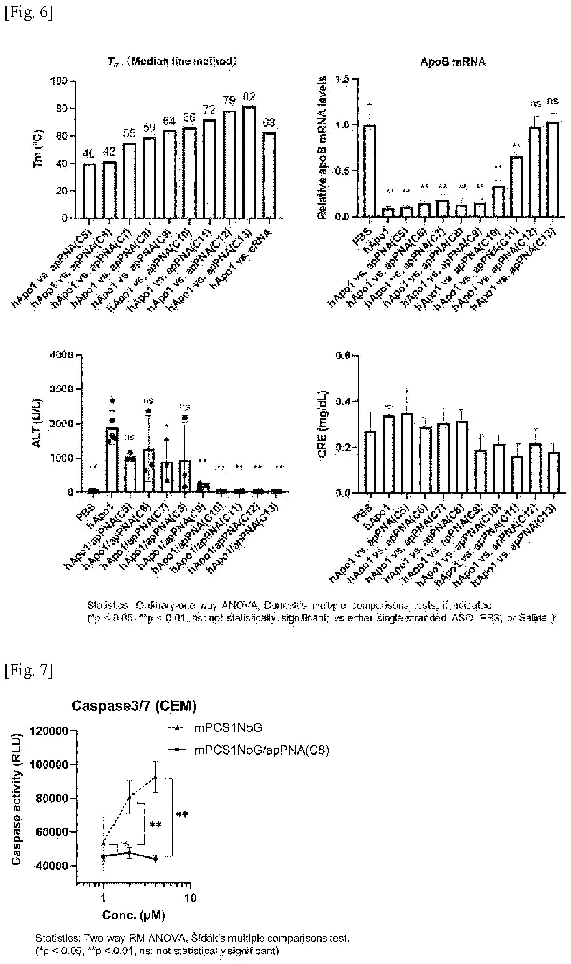

The results are shown in Fig. 6. Fig. 6 shows that even with sequences different from the previous Examples 1 and 3, using PNA with various binding strengths (or toehold lengths) as the complementary strand for single-stranded ASO attenuates hepatotoxicity and nephrotoxicity while also attenuating antisense activity, with increasing thermodynamic stability of the double-strand (or shorter length of the toehold). While no significant difference was observed in some examples due to high data variation since ALT was measured by an enzyme method, overall ALT tended to decrease in accordance with strand length, and since the average ALT value for hApo1/PNA(C5) was lower than the average value for the control (hApo1), it is conjectured that hepatotoxicity was lowered across the full range from hApo1/PNA(C5) to hApo1/PNA(C13). With hApo1/apPNA(C9) in particular, high antisense activity was exhibited while retaining a suitable level for the toxicity markers ALT and CRE. By using BROTHERS duplex with optimal balance between double-strand binding strength, target/ASO binding strength and toehold length it was possible to successfully separate antisense activity and toxicity.

The following were concluded to be the features of the complementary strand (second strand).
1) The double-strand binding strength is in the range of about 40°C to 80°C in terms of Tm (double-strand melting temperature), or:
2) The second nucleic acid strand has a strand length occupying about 35% to 95% of ASO.
3) The length of the toehold is 1 base in length or longer, with an upper limit of about 10 bases.
4) The second nucleic acid strand may be full PNA.
5) The second nucleic acid strand comprises a nucleotide analog and is not a substrate for nucleases such as RNase H.
6) The second nucleic acid strand lacks DDS ligand and the ASO strand has a DDS function.

### [Example 5]

To estimate whether the toxicity-reducing effect of ASO, a feature of the BROTHERS method, could be extrapolated to humans, caspase activity was evaluated using a human liver-derived cell line.

HepG2 cells were seeded in a 96-well plate (5.0 × 10³ cells/well) and incubated for 4 hours at 37°C. After medium removal, 100 µL of medium containing mPCS 1-NoG oligonucleotide (final concentrations: 1, 2 and 4 µM), mPCS 1/apPNA(C8) complex (final concentrations: 1, 2 and 4 µM) and calcium chloride (final concentration: 10 mM) was added to each well and incubated for 72 h at 37°C (CEM method). After adding 100 µL of Caspase-Glo^{®} 3/7 Assay (Promega, Madison, WI) to each well, the plates were incubated for 30 minutes at room temperature, and the luminescence intensity was measured using a multi-mode plate reader.

The results are shown in Fig. 7. Fig. 7 showed concentration-dependent Caspase 3/7 activity with single-stranded ASO, and no change in Caspase activity with BROTHERS complex.

Caspase 3/7 is known as a highly reliable marker for selection of ASO exhibiting strong toxicity in individuals. It is also known that the CEM method using calcium chloride is a transfection method with excellent predictive power for ASO activity in individuals (Nucleic Acids Research, 2015, 43(19), e128). That the concentration-dependent increase in Caspase activity in a human cell line could be suppressed by the BROTHERS method under CEM method conditions strongly suggests that the method can contribute to lowering toxicity in humans as well.

### [Example 6]

For this Example, next-generation sequence analysis was used to evaluate changes in a gene with administration to mice of ASO which exhibits hepatotoxicity and double-strand nucleic acid (mPCS1/apPNA(C8)) with reduced toxicity, as demonstrated in Example 1.

### (Extraction of liver RNA of mouse administered ASO and double-strand nucleic acid)

All animal experiments were conducted with the approval of the Animal Experimentation Committee of Nagasaki University (Nagasaki, Japan). Equimolar mixtures of PCSK9-targeting ASO and PNA were annealed by overnight immersion in hot water followed by gradual cooling. Male mice (7-week-old C57Bl/6J) were purchased from SLC Japan (Tokyo, Japan). ASO or annealed double-strand nucleic acids were subcutaneously administered to mice at a dose of 100 nmol/kg. After 72 hours of a single administration, the mice were anesthetized with isoflurane (Pfizer Japan, Tokyo, Japan) and euthanized to collect their livers and kidneys. The liver tissues were stored overnight in RNAlater^{™} at 4°C and then kept at -20°C until analysis. Total RNA of the livers was extracted using a QuickGene RNA tissue kit SII (FujiFilm Corp.) according to the accompanying manual.

### (Next-generation sequence analysis)

After diluting a total RNA solution 10-fold, it was quantified using a Qubit RNA BR Assay Kit. After quality check using a bioanalyzer (Agilent 2100) (RNA pico), the RNA sample was used to prepare a library with a Collibri 3' mRNA Library prep kit for Illumina Systems (24 reactions) (A38110024, Thermo Fisher Scientific). The final product was analyzed using a Qubit and bioanalyzer. Next-generation sequence analysis was carried out using a NovaSeq 6000 (Illumina).

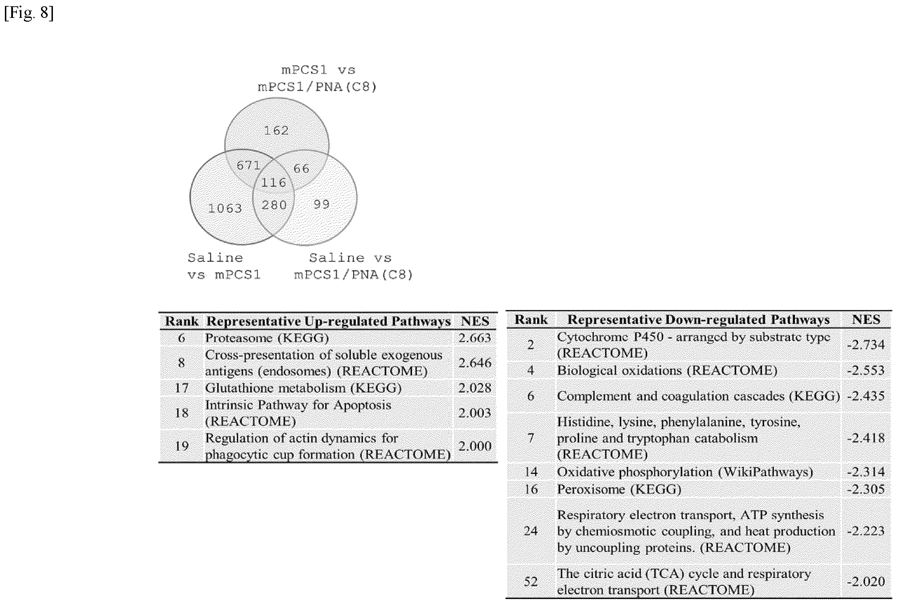

The results are shown in Fig. 8. Fig. 8 shows by next-generation sequence analysis that the significant gene changes with administration was suppressed by about 75%. The results of GO analysis and Pathway analysis strongly indicated a response from gene groups producing oxidative stress (ROS) thought to be associated with tissue damage and gene groups that reduce ROS, as well as a response from gene groups associated with cell death, with single-stranded ASO. When using double-strand nucleic acid, on the other hand, the gene changes were limited. The changed gene groups were thought to be gene groups that generally change even with tissue damage other than in the liver.

### [Example 7]

For this Example, evaluation was made of long-term hepatotoxicity after administration to mice of ASO which exhibits hepatotoxicity and double-strand nucleic acid (mPCS1/apPNA(C8)) with reduced toxicity, as demonstrated in Example 1.

### (Nucleic acid agent synthesis)

An antisense oligonucleotide was synthesized by the same method as Example 1.

### (Synthesis of PNA)

PNA was synthesized by the same method as Example 1.

### (KD activity measurement of ASO in mouse liver)

All animal experiments were conducted with the approval of the Animal Experimentation Committee of Nagasaki University (Nagasaki, Japan). Equimolar mixtures of PCSK9-targeting ASO and PNA were annealed by overnight immersion in hot water followed by gradual cooling. Male mice (7-week-old C57Bl/6J, male) were purchased from SLC Japan (Tokyo, Japan). ASO or annealed double-strand nucleic acids were subcutaneously administered to mice at a dose of 100 nmol/kg. After measuring the body weights on day 3, 6 and 9 after administration, the mice were anesthetized with isoflurane (Pfizer Japan, Tokyo, Japan) and euthanized to collect their livers and kidneys. The liver tissues were stored overnight in RNAlater^{™} at 4°C and then kept at -20°C until analysis. Total RNA of the livers was extracted using a QuickGene RNA tissue kit SII (FujiFilm Corp.) according to the accompanying manual. Reverse transcription was carried out using a High-Capacity RNA-to-cDNA^{™} kit (Thermo Fisher Scientific) with the extracted total RNA as template, and then the expression level of PCSK9 mRNA was analyzed using a CFX real-time PCR system (Bio-Rad). The primer sets used were mPCSK9-F: 5'-TCAGTTCTGCACACCTCCAG-3' and mPCSK9-R: 5'-GGGTAAGGTGCGGTAAGTCC-3' for analysis of mouse PCSK9, and mGAPDH-F: 5'-TGTGTCCGTCGTGGATCTGA-3' and mGAPDH-R: 5'-TTGCTGTTGAAGTCGCAGGAG-3' for analysis of mouse Gapdh as a housekeeping gene, and the KD activity was calculated based on the relative expression level in terms of the difference in Ct value as the difference in expression level.

### (Blood biochemistry test in mice)

Peripheral blood was collected in BD Microtainer tubes (BD, Franklin Lakes, NJ) to separate the serum. The serum AST, ALT and CRE were measured using Fuji dry-chem slide. An aliquot of 10 µL of serum was used for each item measured. The serum total cholesterol levels were measured using LaboAssay^{™} Cholesterol for cell biology (FujiFilm Corp.).

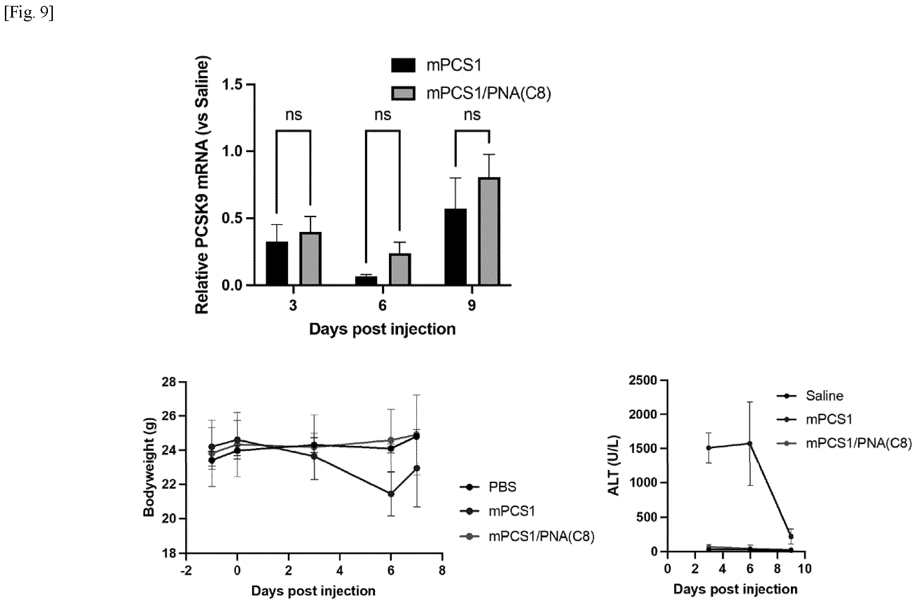

The results are shown in Fig. 9. Fig. 9 showed no increase in deviant enzyme for the prolonged period after administration of the double-strand nucleic acid. However, the decrease in body weight due to toxicity seen upon administration of ASO alone was also notably suppressed in the double-strand nucleic acid administered group.

### [Example 8]

In this Example, evaluation was made of the cytotoxicity of ASO which exhibits hepatotoxicity and double-strand nucleic acid (mPCS1/apPNA(C8)) with reduced toxicity, as demonstrated in Example 1.

### (Nucleic acid agent synthesis)

The mPCS1NoG used in Example 1 was prepared.

### (PNA synthesis)

The mPCS1-apPNA(C8) used in Example 1 was prepared.

### (Cellular experiment)

Transfection of ASO and double-strand nucleic acid was carried out with reference to References 5 and 6. Specifically, ASO and double-strand nucleic acid (mPCS1/apPNA(C8)) were diluted with glucose-free DMEM medium (addition of 10 mM (D+)-Galactose and 9 mM CaCl₂) to a final concentration of 0.01 to 2 µM and added to a 96-well plate. After seeding 5,000 Huh-7 cells diluted with the aforementioned medium into each well, culturing was carried out for 72 hours in an incubator prior to conducting each assay.

### References

5: Nucleic Acids Research, 2015, e128
6: Mol Ther Nucleic Acids, 2021, 957-969

### (Dead cell staining)

After removing medium, the cells were washed with 1 x PBS and then a PI solution diluted to a final concentration of 1.0 µg/mL (Dojindo) was added to each well. After a 15-minutes incubation at 37°C, the staining solution was removed, and cells were washed with 1 x PBS (200 µL). The fluorescence of PI was visualized using a fluorescent microscopy (Carl Zeiss Co.,Ltd.).

### (Cell viability assay)

The medium was replaced with glucose-free DMEM medium (addition of 10 mM (D+)-Galactose, 9 mM CaCl₂) (200 µL/well). Cell Counting Kit-8 (Dojindo) was added at 10 µL/well, and the plates were incubated for 2 hours in an incubator. The absorbance at 450 nm was measured with a microplate reader.

### (LDH assay)

After medium removal, the cells were washed with 1 x PBS and then a Cytotoxicity LDH Assay Kit-WST (Dojindo) was used to measure the LDH activity according to the manufacturer's protocol.

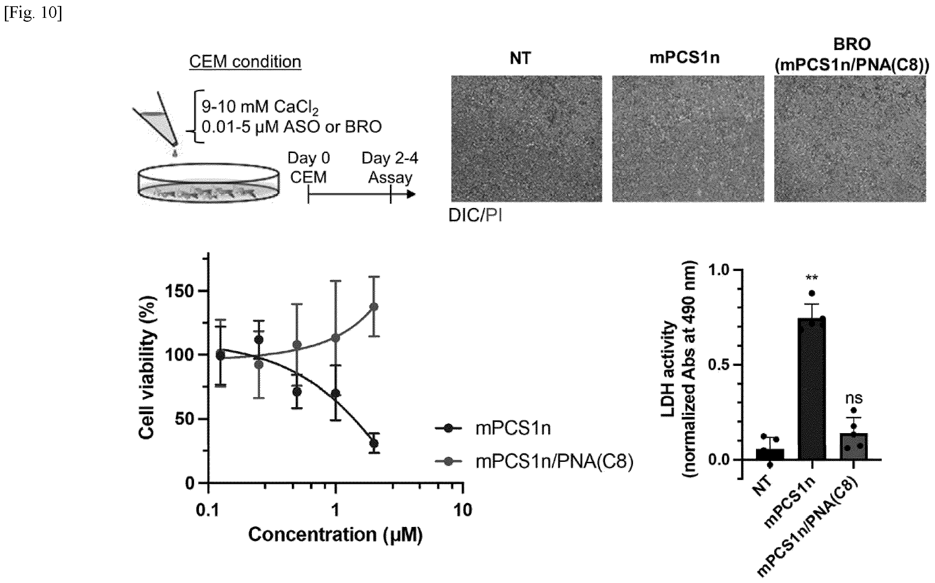

The results are shown in Fig. 10. Fig. 10 shows results for the dead cell staining, cell viability assay and LDH activity evaluation, indicating that concentration-dependent cell death and cell damage were observed *in vitro* with single-stranded ASO, but these phenomena were not seen with double-strand nucleic acid.

### [Example 9]

For this Example, the intracellular dynamics of ASO which exhibits hepatotoxicity and double-strand nucleic acid (mPCS1/apPNA(C8)) with reduced toxicity, as demonstrated in Example 1, was evaluated by imaging using fluorescent-labeled nucleic acid.

### (Synthesis of fluorescent-labeled nucleic acid agent)

ASO with a C6-amino group at the 3'-end was synthesized and purified by the same procedure as Example 1, using amino-on CPG (sigma) as the solid phase. The ASO was dissolved in 1 x PBS to a concentration of 100 µM, and then 5 equivalents of Alexa-Fluor 647 NHS ester (Thermo) was added. The mixture was shaken for 24 hours in the dark. After the reaction, pre-purification was carried out using a desalting column (NAP-5), followed by further purification through reverse-phase HPLC. The HPLC measuring conditions were as follows.

Eluent solution A: 100 mM hexafluoro-2-propanol, 8.6 mM triethylamine (pH 8.3)
Solution B: methanol
Gradient solution B concentration: 5-40% (30 min) (purification)
   5-40% (20 min) (purity confirmation)
Columns: Nacalai ⁵C18-MS-II (10 × 250 mm) (purification)
Nacalai ⁵C18-MS-II (4.6 × 50 mm) (purity confirmation)
Flow rates: 3.0 mL/min (purification)
   1.0 mL/min (purity confirmation)
Column temperature: 60°C
Detection: UV (260 nm)

### (Synthesis of PNA)

The mPCS1-apPNA(C8) used in Example 1 was prepared.

### (Cellular experiment)

This experiment was carried out according to Example 8. ASO and double-strand nucleic acid diluted to a final concentration of 200 nM with DMEM medium (addition of 9 mM CaCl₂) were added to a collagen-coated 35 mm glass-bottom dish. After seeding 10,000 Huh-7 cells diluted with the aforementioned medium into each well, the dish was incubated for 48 hours in an incubator. After medium removal, the cells were washed three times with 1 x PBS and fixed with a 4% paraformaldehyde solution. After incubation for 10 minutes in 0.1% NP-40 (1 x PBS solution) and cell membrane permeabilization, the cells were washed three times with 1 x PBS. After subsequent blocking for 30 minutes with 1% BSA (1 x PBS-T solution), immunostaining was carried out using different antibodies. Cell stain Hoechst 33342 solution was used for staining of the cell nuclei according to the manufacturer's protocol, after which an encapsulant was added dropwise and the intracellular dynamics of the nucleic acid was observed with a confocal microscope.

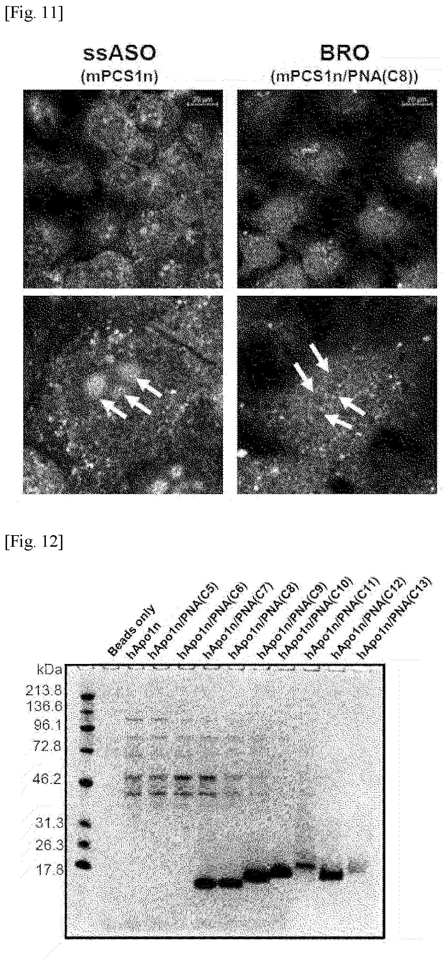

The results are shown in Fig. 11. Fig. 11 shows a comparison of the intracellular dynamics for single-stranded ASO and the double-strand nucleic acid of the invention, with the single-strand ASO exhibiting more migration into the nucleolus compared to the double-strand nucleic acid. With double-strand formation, the manner of localization changes to avoid the nucleolus and mPCS1/apPNA(C8) migrates into the cytoplasm as a double-strand, thus exhibiting different intracellular dynamics than single-stranded ASO.

### [Example 10]

It was examined whether nonspecific protein interaction decreases due to double-strand formation and contributes to lower toxicity even with different ASO sequences.

### (Synthesis of biotin-labeled nucleic acid agent)

The antisense oligonucleotide was synthesized by GeneDesign, Inc. (Osaka, Japan).

### (PNA synthesis)

The PNA sequences used in Example 4 were prepared.

### (Evaluation of bound protein amount by SDS-PAGE)

Mouse livers were homogenized and centrifuged at 10,000 g for 15 minutes and the supernatant was collected, and the protein concentration was determined by the BCA method (TaKaRa BCA Protein Assay Kit, Takara Bio, Inc.). 2 mg of streptavidin beads (Tamagawa Seiki Co., Ltd.) were incubated with 100 µM biotin-labeled ASO for 1 hour at 4°C. After magnetic separation, the mixture was washed 3 times with 100 mM KCl buffer. After magnetic separation and removal of the supernatant, 25 µL of 100 µM PNA (C5-C13) was added respectively. Liver lysate (150 µL) diluted with 100 mM KCl buffer to a protein concentration of 3.0 mg/mL was added to the immobilized beads, and binding reaction was conducted for 2 hours at 4°C. After magnetic separation, the mixture was washed 3 times with 100 mM KCl buffer. ASO or double-strand nucleic acid (1 nmol) was added and the bound protein was competitively eluted by shaking for 5 minutes. After adding SDS sample buffer and heating for 5 minutes at 95°C, the mixture was applied to mPAGE 4-20% Bis-Tris Precast Gel (Merck, Ltd.) and electrophoresed for 40 minutes at 180 V. The gel was stained with CBB for 1 hour, after which it was decolorized for 2 hours with CBB decolorizing solution. A CBB stained gel image was obtained using a ChemiDoc^{™} Touch imaging system (Bio-Rad).

The results are shown in Fig. 12. As shown in Fig. 12, it was observed that the protein binding amount was drastically reduced by introduction of the complementary strand (brother strand) even with ASO (hApo1) having different sequences. The bound protein was also shown to gradually decrease with extension of the complementary strand, suggesting a relationship between bound protein amount and toxicity.

### [Example 11]

It was tested whether the commonly used complementary strand in HDO could be used to reduce the toxicity of ASO.

### (Nucleic acid agent synthesis)

The antisense oligonucleotide of Example 1 (mPCSK9-2590-BNA (14); mPCS1) was prepared by the same method as Example 1.

### (Synthesis of complementary strand)

The complementary strand (HDO(14)-mPCS1) was synthesized by GeneDesign, Inc. (Osaka, Japan). The sequence of the complementary strand was the following. G(M)^G(M)^A(M)^GAACUUGG^U(M)^G(M)^U(M) (SEQ ID NO: 40) ^: Phosphorothioate (PS) bond, N(M): 2'-OMe RNA, uppercase: RNA

### (KD activity measurement of ASO in mouse liver)

All animal experiments were conducted with the approval of the Animal Experimentation Committee of Nagasaki University (Nagasaki, Japan). Equimolar mixtures of PCSK9-targeting ASO and HDO were annealed by overnight immersion in hot water followed by gradual cooling. Male Mice (7-week-old C57Bl/6J) were purchased from SLC Japan (Tokyo, Japan). ASO or annealed HDO were subcutaneously administered to mice at a dose of 400 nmol/kg. After 72 hours of a single administration, the mice were anesthetized with isoflurane (Pfizer Japan, Tokyo, Japan) and euthanized to collect their livers and kidneys. The liver tissues were stored overnight in RNAlater^{™} at 4°C and then kept at -20°C until analysis. The total RNA of the livers was extracted using a QuickGene RNA tissue kit SII (FujiFilm Corp.) according to the accompanying manual. Reverse transcription was carried out using a High-Capacity RNA-to-cDNA^{™} kit (Thermo Fisher Scientific) with the extracted total RNA as template, and then the expression level of PCSK9 mRNA was analyzed using a CFX real-time PCR system (Bio-Rad). The primer sets used were mPCSK9-F: 5'-TCAGTTCTGCACACCTCCAG-3' and mPCSK9-R: 5'-GGGTAAGGTGCGGTAAGTCC-3' for analysis of mouse PCSK9, and mGAPDH-F: 5'-TGTGTCCGTCGTGGATCTGA-3' and mGAPDH-R: 5'-TTGCTGTTGAAGTCGCAGGAG-3' for analysis of mouse Gapdh as a housekeeping gene, and the KD activity was calculated based on the relative expression level in terms of the difference in Ct value as the difference in expression level.

### (Blood biochemistry test in mice)

Peripheral blood was collected in BD Microtainer tubes (BD, Franklin Lakes, NJ) to separate the serum. Measurement of ALT was performed by Oriental Yeast Co., Ltd.. The measurement was performed using an L-type Wako ALT·J2 (FujiFilm Corp.).

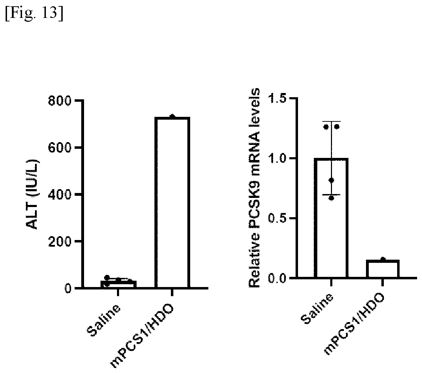

The results are shown in Fig. 13. Fig. 13 shows that knockdown of PCSK9 mRNA, but also a significant increase in ALT, was observed when using HDO(14)-mPCS1 which is similar to HDO known as a complementary strand. It has been disclosed that HDO has an unmodified nucleic acid region in the complementary strand, which is important for decomposition by endosomes and lysosomes to a single-strand. BROTHERS, on the other hand, migrates into cells as a double-strand and functions, as mentioned above, suggesting a connection between the intracellular dynamics and low toxicity characteristic of BROTHERS.

### [Example 12]

It was examined *in vitro* whether or not activation of the Caspase 3/7 pathway as generally seen with highly toxic ASO can be suppressed by double-strand formation.

### (Nucleic acid agent synthesis)

The mPCS1NoG used in Example 1 was prepared.

### (Synthesis of PNA)

The mPCS1-apPNA(C8) used in Example 1 was prepared.

### (Cellular experiment)

Transfection of ASO and double-strand nucleic acid was carried out by the same method as Example 8. ASO and double-strand nucleic acid (mPCS1/apPNA(C8)) were diluted with glucose-free DMEM medium (addition of 10 mM (D+)-Galactose and 9 mM CaCl₂) or low-glucose DMEM medium (addition of 9 mM CaCl₂) to a final concentration of 2 µM and added to a 96-well plate. After seeding 5,000 Huh-7 cells diluted with the aforementioned medium into each well, culturing was carried out for 48 hours in an incubator prior to conducting each assay.

### (Caspase assay)

After medium replacement, Caspase 3/7, Caspase 8 and Caspase 9 activation were measured using Caspase-Glo (registered trademark) 3/7 Assay Systems, Caspase-Glo (registered trademark) 8 Assay Systems and Caspase-Glo (registered trademark) 9 Assay Systems (Promega), according to the manufacturer's protocol.

### (Dead cell staining)

The dead cells were stained by the same procedure as Example 8 and observed with a fluorescent microscope.

### (Observation of mitochondrial membrane potential)

A JC-1 MitoMP Detection Kit (Dojindo) was used for staining according to the manufacturer's protocol, and the cells were observed with a fluorescent microscope (Green: Ex 488 nm/Em 500-550 nm, Red: Ex 561 nm/Em, 560-610 nm).

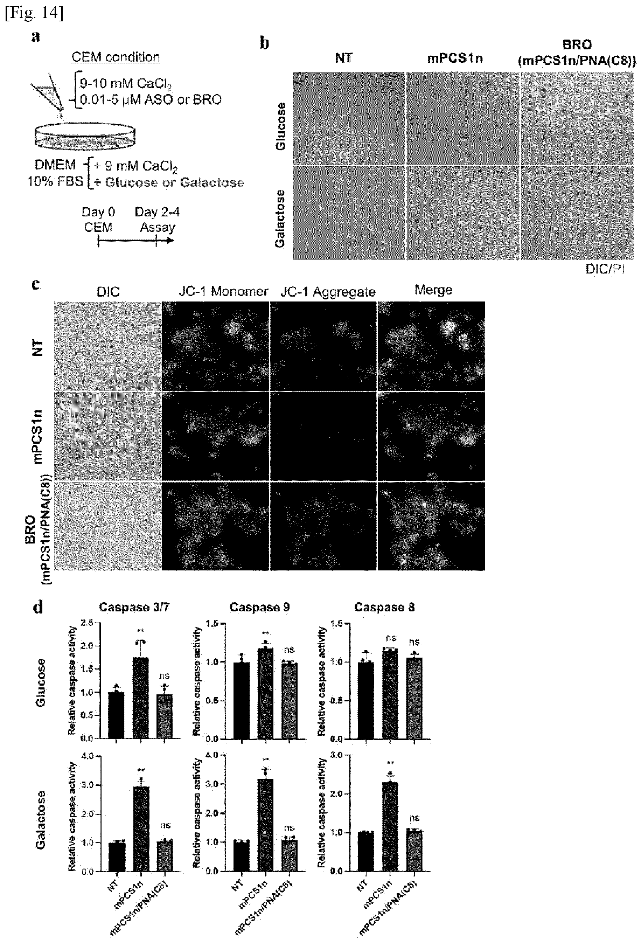

The results are shown in Fig. 14. Fig. 14 confirmed notable cytotoxicity with single-stranded ASO by changing to galactose medium. Galactose medium is generally used to investigate mitochondrial toxicity of drugs, and single-stranded ASO may be involved in mitochondrial toxicity. Double-strand nucleic acid did not exhibit activation of Caspase 3/7, 9 and 8 often seen with single-stranded ASO and suppressed mitochondrial toxicity, exhibiting no significant cytotoxicity. These *in vitro* comparison results matched well with the difference in toxicities of single-stranded ASO and double-strand nucleic acid observed *in vivo.* Since mitochondrial toxicity and Caspase 3/7 activation are not limited to liver cells but are universal in the mechanism of cell death (see Reference 7), this double-strand nucleic acid technology that can suppress the cell death pathway is effective as a method for general lowering of cytotoxicity, without limitation to hepatotoxicity.

### References

7: Mol Ther Nucleic Acids., 2018, 45-54.

### [Example 13]

The strand-exchange reaction rates and off-target effects of double-strand nucleic acids for the off-target genes containing mismatches listed in Table 6 were evaluated. SEQ ID NO: 41 to 45 in the Sequence Listing are the sequences of Table 6 in order from the top.

**Table 6**

| Position | | -1 | 1 | 2 | 3 | 4 | 5 | 6 | 7 | 8 | 9 | 10 | 11 | 12 | 13 | |
|---|---|---|---|---|---|---|---|---|---|---|---|---|---|---|---|---|
| Target mRNAs | Sequence | | | | | | | | | | | | | | | |
| hApo1nF | 5' | F | A | A | t | g | g | c | c | a | g | c | T | T | G | 3' |
| ApoB | 3' | Q | rU | rU | rA | rC | rC | rG | rG | rU | rC | rG | rA | rA | rC | 5' |
| Copg | 3' | Q | rU | rU | rA | rC | rC | rG | rG | rG | rC | rG | rA | rA | rC | 5' |
| Mast2 | 3' | Q | rU | rU | rA | rC | rC | rG | rG | rU | rG | rG | rA | rA | rC | 5' |
| Hltf | 3' | Q | rU | rU | rA | rC | rC | rG | rG | rU | rU | rG | rA | rA | rC | 5' |

| | | | | | | | | | | | | | | | | |
|---|---|---|---|---|---|---|---|---|---|---|---|---|---|---|---|---|
| N: LNA, n: DNA, F: TAMRA, Q: BHQ2, rN: RNA | | | | | | | | | | | | | | | | |

### (Nucleic acid agent synthesis)

The nucleic acids were synthesized by GeneDesign, Inc. (Osaka, Japan).

### (PNA synthesis)

The hApo1-apPNA(C9) used in Example 4 was prepared.

### (Evaluation of strand exchange rate)

Each BHQ2-labeled 100 nM RNA (50 µL) was added to sample solution (50 µL) containing final concentrations of 10 mM phosphate buffer (pH 7.0), 100 mM sodium chloride, 0.1 mM ethylenediaminetetraacetic acid and 100 nM hApo1F and hApo1F/apPNA(C9), and the time-dependent change in attenuating fluorescence intensity was measured.

### (Cellular experiment)

Transfection of ASO and double-strand nucleic acid was carried out by the same method as Example 8. ASO and double-strand nucleic acid (mPCS 1/apPNA(C8)) were diluted with low-glucose DMEM medium (addition of 9 mM CaCl₂) to a final concentration of 1 µM and added to a 96-well plate. After seeding 10,000 Huh-7 cells diluted with the aforementioned medium into each well, culturing was carried out for 24 hours in an incubator. A SuperPrep (registered trademark) II Cell Lysis & RT Kit for qPCR (Toyobo) was used to prepare cDNA from the cell lysate according to the manufacturer's protocol. The expression levels of mRNA of ApoB and the off-target genes listed in Table 6 were analyzed with a CFX real-time PCR system (Bio-Rad). The primer sets used were hApoB-F: 5'-TTCTCAAGAGTTACAGCAGATCCA-3' (SEQ ID NO: 58) and hApoB-R: 5'-TGGAAGTCCTTAAGAGCAACTAACA-3' (SEQ ID NO: 59) for analysis of human ApoB, hCopg-F: 5'-CCATGCATTGGGAGTCCTGT-3' (SEQ ID NO: 60) and hCopg-R: 5'-ACTGGCAATTCGGATCAGCA-3' (SEQ ID NO: 61) for analysis of human Copg, hMast2-F: 5'-TGACTTTCGCTGAGAACCCC-3' (SEQ ID NO: 62) and hMast2-R: 5'-TCTTCAGCAGAGTGGCACAG-3' (SEQ ID NO: 63) for analysis of human Mast2, hHltf-F: 5'-CCTGTGCCGTTTTCTTGCTC-3' (SEQ ID NO: 64) and hHltf-R: 5'-CCCTGCAGAAAGGTCTTGGT-3' (SEQ ID NO: 65) for analysis of human Hltf, and hGAPDH-F: 5'-GCACCGTCAAGGCTGAGAAC-3' (SEQ ID NO: 66) and hGAPDH-R: 5'-TGGTGAAGACGCCAGTGGA-3' (SEQ ID NO: 67) for analysis of human Gapdh as a housekeeping gene, and the KD activity was calculated based on the relative expression level in terms of the difference in Ct value as the difference in expression level.

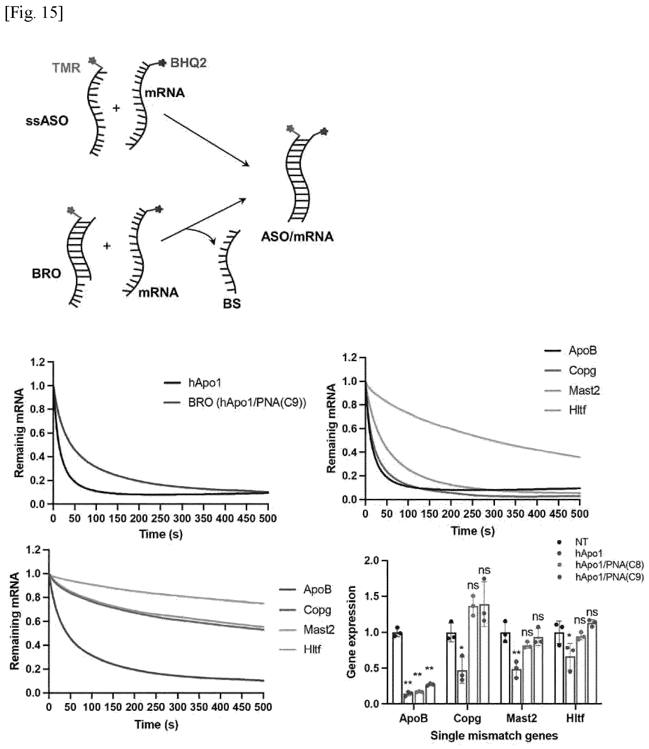

The results are shown in Fig. 15. Fig. 15 shows that with the single-stranded nucleic acid, the off-target genes were bound at about the same rate as the on-target genes, whereas with the BROTHERS-type double-strand nucleic acid, the strand-exchange reaction with off-target genes was significantly lower compared to on-target genes. Unintended knockdown of off-target genes was also eliminated in cultured cells as well, in correlation with the strand-exchange reaction rate in aqueous solution. The hybridization-dependent off-target suppressing effect of the double-strand nucleic acid is thought to be due to the characteristic structure of BROTHERS.

### [Example 14]

It was verified whether or not using parallel PNA (pPNA) for longer ASO can allow design of double-strand nucleic acid having suitable strand lengths and binding affinity and with similarly lower toxicity. The ASO and pPNA used in this Example are shown in Table 7. SEQ ID NO: 46 to 54 in the Sequence Listing are the sequences of Table 7 in order from the top. The sequences listed as SEQ ID NO: 46 to 48 are antisense strand sequences, and the sequences listed as SEQ ID NO: 49 to 54 are complementary strand sequences (N-terminus → C-terminus).

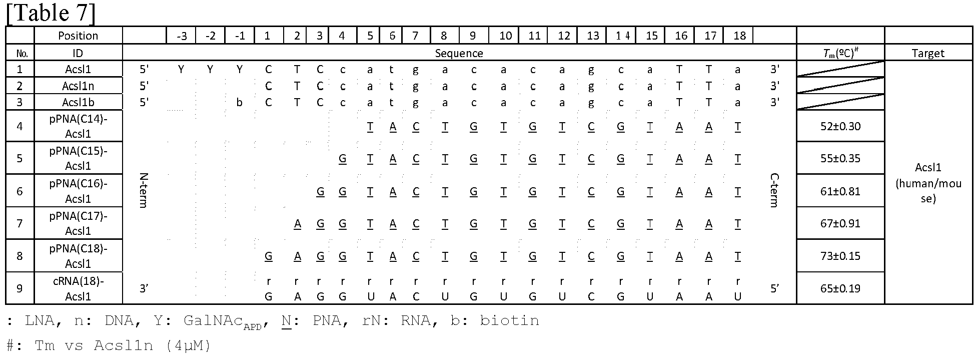

### (Nucleic acid agent synthesis)

The Acsl1 and Acsl1n in Table 7 were synthesized in the same manner as Example 1. The biotin-labeled ASO was synthesized by GeneDesign, Inc. (Osaka, Japan).

### (PNA synthesis)

Each PNA in Table 7 was synthesized in the same manner as Example 1.

### (Melting temperature (Tₘ) measurement (evaluation of duplex forming ability))

The oligonucleotide solution was prepared to a final concentration of 4 µM in a 10 mM sodium phosphate buffer (pH 7.0) containing 100 mM NaCl and 0.1 mM ethylenediaminetetraacetic acid. The mixture was annealed by heating at 95°C for 3 minutes and then cooled at a rate of 1°C per minute to 20°C. The melting temperature (Tm) was estimated using the software provided with the TMSPC-8, defined as the temperature at which the formed duplexes were half-dissociated. The temperature-dependent change in optical absorption was recorded at 260 nm, with a scan rate of 0.5°C per minute from 4°C to 90°C. Each averaged *T*ₘ value was determined as the intersection point between the absorption versus temperature curves.

### (KD activity measurement of ASO in mouse liver)

All animal experiments were conducted with the approval of the Animal Experimentation Committee of Nagasaki University (Nagasaki, Japan). Equimolar mixtures of Acsl1-targeting ASO and PNA were annealed by overnight immersion in hot water followed by gradual cooling. Male Mice (7-week-old C57Bl/6J) were were purchased from SLC Japan (Tokyo, Japan). ASO or annealed were subcutaneously administered to mice at a dose of 100 nmol/kg. After 96 hours of a single administration, the mice were anesthetized with isoflurane (Pfizer Japan, Tokyo, Japan) and euthanized to collect their livers and kidneys. The liver tissues were stored overnight in RNAlater^{™} at 4°C and then kept at -20°C until analysis. The total RNA of the livers was extracted using a QuickGene RNA tissue kit SII (FujiFilm Corp.) according to the accompanying manual. Reverse transcription was carried out using a High-Capacity RNA-to-cDNA^{™} kit (Thermo Fisher Scientific) with the extracted total RNA as template, and then the expression level of Acsl1 mRNA was analyzed using a CFX real-time PCR system (Bio-Rad). The primer sets used were mAcsl1-F: 5'-AGGTGCTTCAGCCCACCATC-3' (SEQ ID NO: 68) and mAcsl1-R: 5'-AAAGTCCAACAGCCATCGCTTC-3' (SEQ ID NO: 69) for analysis of mouse Acsl1, and mGAPDH-F: 5'-TGTGTCCGTCGTGGATCTGA-3' and mGAPDH-R: 5'-TTGCTGTTGAAGTCGCAGGAG-3' for analysis of mouse Gapdh as a housekeeping gene, and the KD activity was calculated based on the relative expression level in terms of the difference in Ct value as the difference in expression level.

### (Blood biochemistry test in mice)

Serum harvested from inferior vena cava blood was used to measure AST, ALT and CRE using FujiDryChem. A 10 µL portion of serum was used for each item measured. The serum total cholesterol concentration was measured using LaboAssay^{™} Cholesterol for cell biology (FujiFilm Corp.).

### (Evaluation of bound protein amount by SDS-PAGE)

Mouse livers were homogenized and centrifuged at 10,000 g for 15 minutes and the supernatant was collected, and the protein concentration was determined by the BCA method (TaKaRa BCA Protein Assay Kit, Takara Bio, Inc.). 2 mg of streptavidin beads (Tamagawa Seiki Co., Ltd.) were incubated with 100 µM biotin-labeled ASO for 1 hour at 4°C. After magnetic separation, the mixture was washed 3 times with 100 mM KCl buffer, after which 25 µL of 100 µM PNA (C14-18) was added and dispersed. Liver lysate (150 µL) diluted with 100 mM KCl buffer to a protein concentration of 3.0 mg/mL was added to the immobilized beads, and binding reaction was conducted for 2 hours at 4°C. After magnetic separation, the mixture was washed 3 times with 100 mM KCl buffer, and then ASO or double-strand nucleic acid (1 nmol) was added and the bound protein was competitively eluted by shaking for 5 minutes. After adding SDS sample buffer and heating for 5 minutes at 95°C, the mixture was applied to mPAGE 4-20% Bis-Tris Precast Gel (Merck, Ltd.) and electrophoresed for 40 minutes at 180 V. The gel was stained with CBB for 1 hour, after which it was decolorized for 2 hours with CBB decolorizing solution. A CBB stained gel image was obtained using a ChemiDoc^{™} Touch imaging system (Bio-Rad).

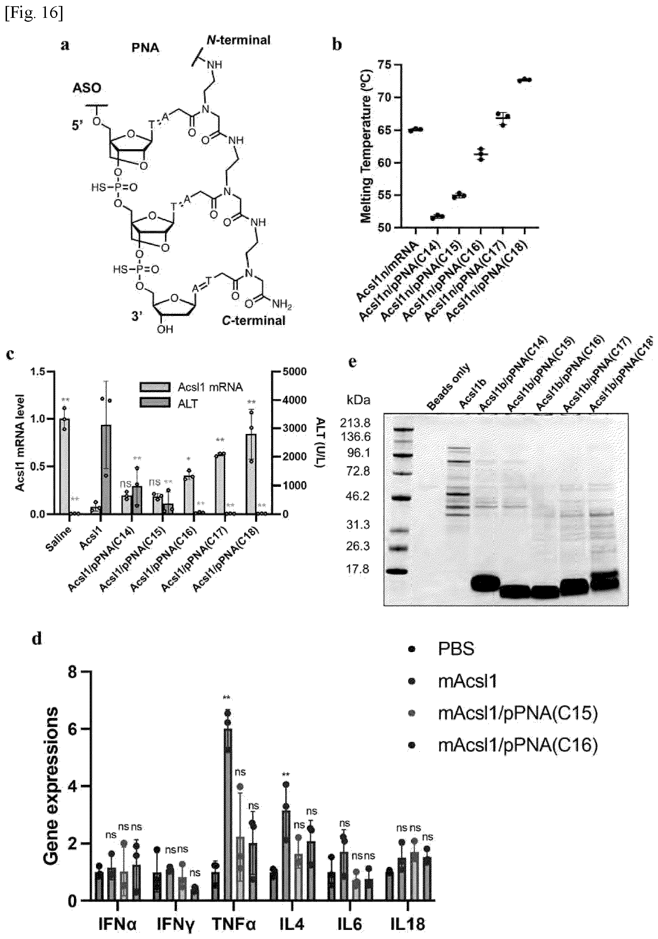

The results are shown in Fig. 16. Fig. 16 confirmed that designing pPNA of suitable strand length for long ASO strands suppresses increase in deviant liver enzymes while retaining knockdown activity, and also significantly suppresses expression of cytokines. The amounts of bound proteins attributable to toxicity were also drastically reduced by double-strand formation, similar to the other Examples. These results indicate that by adjusting the balance between binding affinity and toehold length by physicochemical and organic chemistry methods when designing brother strands for ASO with different lengths, sequences and chemical properties, it is possible to suppress the toxicity exhibited by different ASO (tissue damage or (natural) immune response provoked by interaction between cellular components and blood components).

### [Example 15]

### (Nucleic acid agent synthesis)

Each hApo1 in Table 8 was synthesized in the same manner as Example 1.

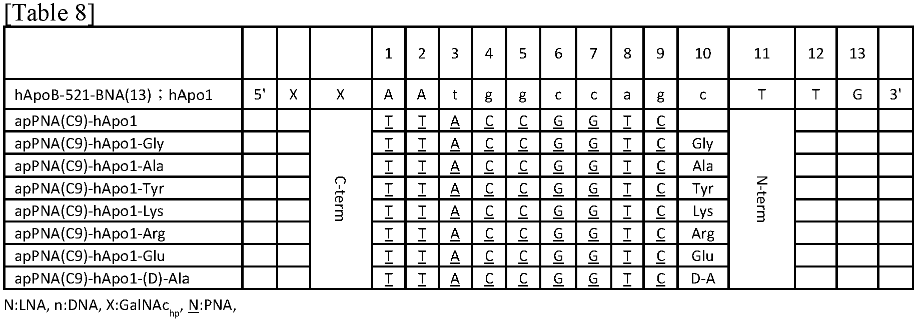

### (Synthesis of PNA)

Each PNA in Table 8 was synthesized in the same manner as Example 1.

### (Melting temperature (Tm) measurement (evaluation of double-strand forming ability))

The oligonucleotide solution was prepared to a final concentration of 4 µM in a 10 mM sodium phosphate buffer (pH 7.0) containing 100 mM NaCl and 0.1 mM ethylenediaminetetraacetic acid. The mixture was annealed by heating at 95°C for 3 minutes and then cooled at a rate of 1°C per minute to 20°C. The melting temperature (Tm) was estimated using the software provided with the TMSPC-8, defined as the temperature at which the formed duplexes were half-dissociated. The temperature-dependent change in optical absorption was recorded at 260 nm, with a scan rate of 0.5°C per minute from 4°C to 90°C. Each averaged *T*ₘ value was determined as the intersection point between the absorption versus temperature curves.

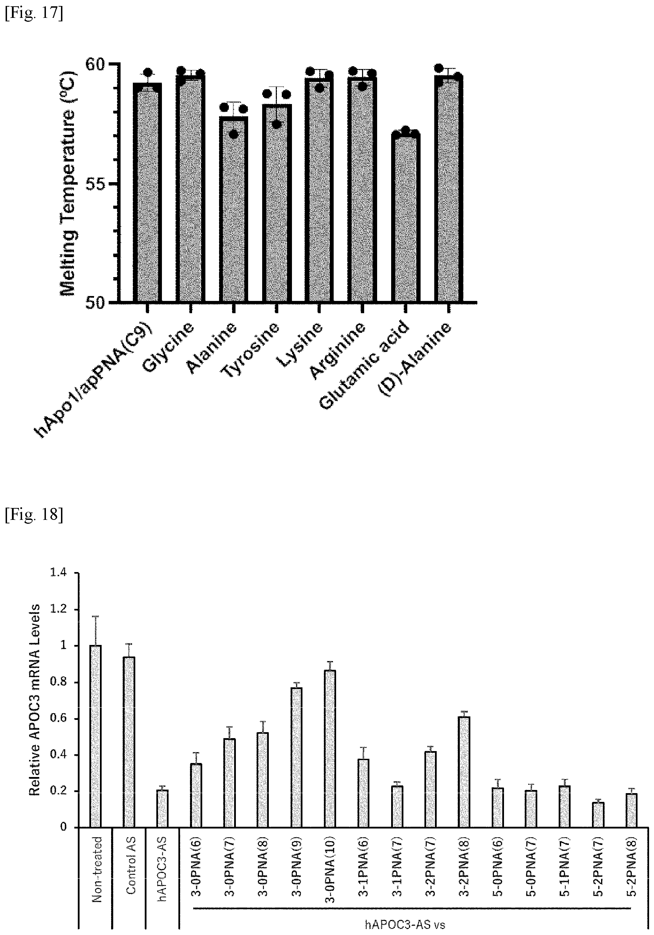

The results are shown in Fig. 17. Fig. 17 verified that it was possible to perturb the binding strength when different amino acids were introduced at the ends of the complementary strand (brother strand) for single-stranded ASO. By chemical modification or monomer modification of the PNA strand, it was possible to achieve optimization to double-strand nucleic acid with the desired binding affinity and toehold length, similar to the approach using parallel PNA in Example 14.

### [Example 16]

### (Nucleic acid synthesis)

The Control-AS was synthesized by GeneDesign, Inc., the hAPOC3-AS was synthesized by Nitto Denko Corp., and the PNA-containing oligonucleotides (Table 9) were synthesized to Fasmac Corp., all on request. The sequences of the hAPOC3-AS (antisense strand), 3-0PNA(10) (complementary strand) and Control-AS in Table 9 having 10 or more base pairs are SEQ ID NO: 55 to 57 in the Sequence Listing.

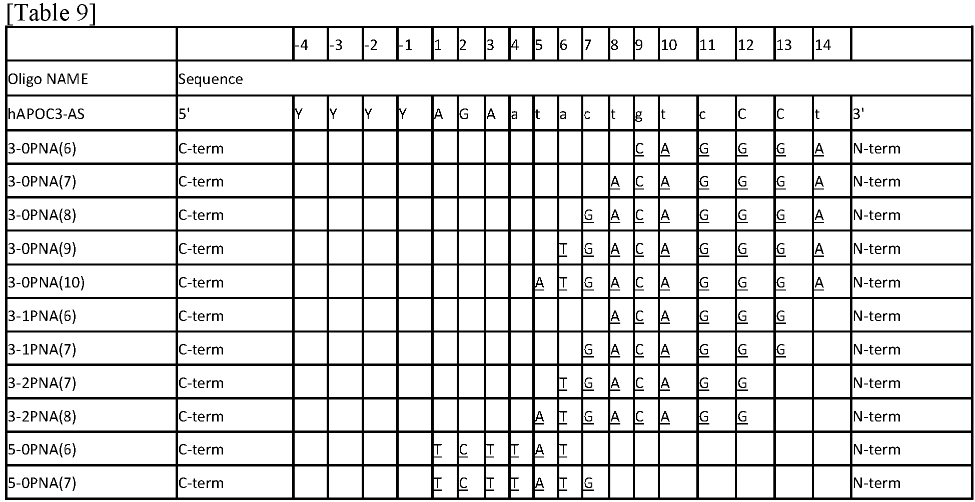

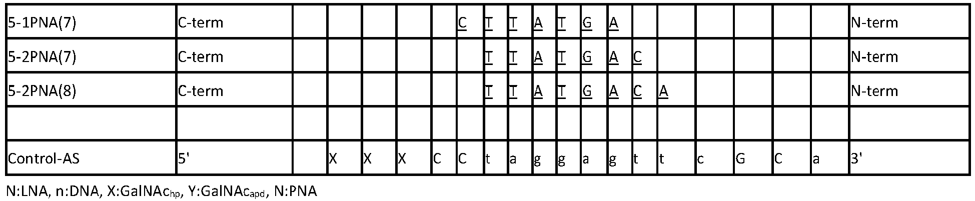

### (Evaluation of activity using human liver cancer cell line Huh-7)

While seeding Huh7 cells suspended in low-glucose DMED medium (1.0 × 10⁴ cells/well) with addition of calcium chloride (final concentration: 9 mM) in a 96-well plate, there were added Control-AS and hAPOC3-AS (final concentration: 25 nM), and hAPOC3-AS vs PNA (hAPOC3-AS, 3-0PNA(6), 3-0PNA(7), 3-0PNA(8), 3-0PNA(9), 3-0PNA(10), 3-1PNA(6), 3-1PNA(7), 3-2PNA(7), 3-2PNA(8), 5-0PNA(6), 5-0PNA(7), 5-1PNA(7), 5-2PNA(7) and 5-2PNA(8)) complex (final concentration: 25 nM) to every 4 wells, and the cells were incubated for 24 h at 37°C. A SuperPrep (registered trademark) II Cell Lysis & RT Kit for qPCR (Toyobo) was used to create cDNA, and a Taqman^{™} probe (Thermo Fisher Scientific, Hs02758991 _g1 GAPDH VIC PL, Hs00163644_m1 APOC3 FAM) was used for quantitation of the APOC3 mRNA using GAPDH as a housekeeping gene, using a real-time PCR system (Applied Biosystems).

The results are shown in Fig. 18. Fig. 18 confirmed that adjustment of the brother strand length and relative position on the antisense strand, as well as the lengths, positions and number of toeholds, increases or decreases knockdown activity of target mRNA. This indicated that adjustment or optimization of the length of the brother strand and the position on the antisense strand, as well as the lengths, positions and number of toeholds is effective for maximizing the effect of BROTHERS.

### INDUSTRIAL APPLICABILITY

The present invention provides novel double-strand nucleic acid. The nucleic acid can be administered to cells or an individual to regulate gene expression with reduced toxicity, allowing it to be used particularly as a therapeutic agent for a disease.

The present application claims priority based on Japanese Patent Application No. 2021-198890 (filed in Japan on December 7, 2021), the entirety of the disclosure of which is incorporated herein by reference.

[Sequence Listing]

## Claims

1. An antisense oligonucleotide complex which comprises an antisense oligonucleotide and a complementary strand comprising a sequence complementary to the antisense oligonucleotide,
wherein the antisense oligonucleotide has a toehold with a single-stranded structure, and
the complementary strand has nuclease resistance across its full length,
the antisense oligonucleotide complex having an antisense effect and reduced toxicity.

2. The antisense oligonucleotide complex according to claim 1, wherein the length of the toehold is 1 to 10 bases in length.

3. The antisense oligonucleotide complex according to claim 1 or 2, wherein all of the nucleotides composing the complementary strand are non-natural nucleotides.

4. The antisense oligonucleotide complex according to any one of claims 1 to 3, wherein at least one of the nucleotides composing the complementary strand is a PNA nucleotide.

5. The antisense oligonucleotide complex according to any one of claims 1 to 4, wherein all of the nucleotides composing the complementary strand are PNA nucleotides.

6. The antisense oligonucleotide complex according to any one of claims 1 to 5, wherein:
(1) the melting temperature (Tm) of the double-stranded nucleic acid is 40°C to 80°C, and/or
(2) the length of the complementary strand is 35% to 95% of the length of the antisense oligonucleotide.

7. The antisense oligonucleotide complex according to any one of claims 1 to 6, wherein:
(1) the Tm is 50°C to 70°C, and/or
(2) the length of the complementary strand is 60% to 80% of the length of the antisense oligonucleotide.

8. The antisense oligonucleotide complex according to any one of claims 1 to 7, wherein at least one functional molecule is bonded to either or both the antisense oligonucleotide strand and complementary strand.

9. The antisense nucleotide complex according to claim 8, wherein the functional molecule is bonded only to the antisense oligonucleotide.

10. The antisense nucleotide complex according to any one of claims 1 to 9, wherein the antisense oligonucleotide targets mRNA selected from the group consisting of PCSK9 mRNA, ApoB mRNA, AcsL1 mRNA and ApoC3 mRNA.

11. A pharmaceutical composition comprising the antisense oligonucleotide complex according to any one of claims 1 to 10.

12. A composition for regulating expression of a target gene, comprising the antisense oligonucleotide complex according to any one of claims 1 to 10.

13. A method for producing an antisense oligonucleotide complex with reduced toxicity, comprising:
(1) a step of preparing an antisense oligonucleotide,
(2) a step of preparing a complementary strand in which the antisense oligonucleotide has a toehold with a single-stranded structure when a double-strand has been formed with the antisense oligonucleotide, and wherein
the complementary strand has nuclease resistance across the full length, and
(3) a step of annealing the antisense oligonucleotide prepared in step (1) and the complementary strand prepared in step (2).

14. The method according to claim 13, wherein the length of the toehold is 1 to 10 bases in length.

15. A method for designing an antisense oligonucleotide complex with reduced toxicity, comprising:
(1) a step of selecting an antisense oligonucleotide, and
(2) a step of designing a complementary strand in which the antisense oligonucleotide has a toehold with a single-stranded structure when a double-strand has been formed with the antisense oligonucleotide, and wherein
the complementary strand has nuclease resistance across the full length.

16. The method according to claim 15, wherein the length of the toehold is 1 to 10 bases in length.
